# EUROPEAN PATENT APPLICATION

(11) **EP 2 450 708 A2**
(43) Date of publication of application: **09.05.2012**
(21) Application number: 11007496.0
(22) Date of filing: 11.04.2005
(51) Int. Cl.: G01N 33/50, C40B 30/04

(54) **Protein-Protein interactions for pharmacological profiling**

(30) Priority: 12.04.2004 US 560975 P
(62) Divisional of application: 05735396.3
(71) Applicant: Odyssey Thera, Inc., San Ramon, CA 94583 (US)
(72) Inventor: Westwick, John K., San Ramon, CA 94583 (US); Keon, Brigitte, Castro Valley, CA 94552 (US); Mac Donald, Marnie, Pleasanton, CA 94566 (US); Michnick, Stephen William Watson, Montreal, Quebec H2J 3C8 (CA)
(74) Representative: Schüssler, Andrea

(57) **Abstract**

The present invention provides methods for performing pharmacological profiling of a chemical compound, in particular to improve drug safety and efficacy at an early stage in the drug development process. The chemical compound may be a test compound, drug lead, known drug or toxicant. The compound is profiled against a panel of assays. Preferred embodiments of the invention include high-content assays for protein-protein interactions. The compositions and methods of the invention can be used to identify pathways underlying drug efficacy, safety, and toxicity; and to effect attrition of novel compounds with undesirable or toxic properties. The compositions and methods of the invention can also be used to identify new uses of therapeutic agents, to screen libraries of chemical compounds, to perform lead optimization, and to perform studies of structure-activity relationships in the context of intact cells. The compositions and methods of the invention can be applied to any test compound, drug, drug target, pathway, and therapeutic indication.

## Description

This application claims the benefit of priority of U.S. provisional No. 60/560,975 filed April 12, 2004. This application is a continuation-in-part of pending U.S. Serial No. 09/968,864 filed October 3, 2001; which claims the benefit of priority of U.S provisional No. 60/237,690 filed October 5, 2000. This Application is also a continuation-in-part of pending U.S. Application Serial No. 10/353,090 filed January 29, 2003; which application is a continuation of pending U.S. application No. 10/154,758 filed May 24, 2002; which application is a continuation of U.S. Serial No. 09/499,464 filed February 7, 2000; and now U.S. Patent No. 6,428,951; which is a continuation of U.S. Serial No. 09/017,412 filed February 2, 1998; and now U.S. Patent No. 6,270,964. The entire contents of all those patents, applications and provisional applications are incorporated by reference herein.

### BACKGROUND OF THE INVENTION

The concept of selective drugs has dominated the discovery and development of new therapeutics over the last century. A molecule may be a potentially useful therapeutic agent if it can be shown to selectively effect a change in cellular physiology by acting in a desired way on a target within the biochemical pathways that underlie the physiological process of interest. However, even an exquisitely selective chemical compound that binds to a therapeutic target may have completely unexpected or 'off-pathway' effects when it contacts a living cell. Such effects may result in expensive pre-clinical and clinical failures. For the purposes of this invention, we define 'off-pathway' activity as any activity of a compound on a pathway other than the intended target of the compound.

Identifying mechanisms of action of drugs, and their off-pathway activities, is not achievable with enzyme assays because it is not feasible to set up an assay for each of the tens of thousands of proteins representing the biological milieu. In order to assess the mode of action of a drug within the complex biochemical pathways that make up a living cell, one needs a way to probe the pathways directly. Therefore, we sought a general strategy for pharmacological profiling. In particular we sought to determine whether quantitative measures of protein-protein interactions could be used to profile drug activity on a large scale.

The background for the present invention is as follows. Binding of agonists to receptors induces a cascade of intracellular events mediated by other signaling molecules. Conceptually, such signaling cascades involve the regulated association and dissociation of proteins within macromolecular complexes. Further, the assembly and disassembly of protein-protein complexes occurs dynamically upon addition of an agonist, antagonist or inhibitor of a pathway. Finally, the assembly and disassembly of specific protein-protein complexes occurs at particular subcellular locations such as the cell membrane, cytoplasm, nucleus, mitochondria, or other compartments within the cell.

Most cell-based pharmacological profiling to date has been performed with DNA microarrays (gene chips). DNA microarrays have spawned the field of toxicogenomics, which involves the use of complex populations of mRNA to understand toxicitiy. Cells, or whole animals, are treated with drugs; messenger RNA is isolated from the cell or tissue; and the gene expression patterns of the mRNA in the absence and presence of a drug are compared. Such transcriptional profiling can reveal differences between compounds, where the compounds affect the ultimate transcriptional activity of one or more pathways. Identifying specific pathways that are stimulated or repressed in response to specific conditions or treatments is a useful way to begin to unravel the cellular mechanisms of disease and of drug response. However, changes in the level of individual mRNA molecules will not always correlate directly with the level or activity of the corresponding protein at a single point in time. Furthermore, many proteins undergo numerous post-translational modifications and protein interactions, which may affect the functions and activities of proteins within a tissue or cell. Thus, simply identifying all of the mRNA species present and the levels at which they are present at a particular time, may not yield the complete picture of a particular drug. Finally, a targeted drug may affect the transcription of dozens of genes, making interpretation of the results of gene chip experiments an arduous task.

The regulation of cellular responses is mediated by a certain "threshold" number of molecular interactions that eventually reach the cell nucleus and induce the genetic apparatus of the cell to synthesize newly expressed proteins in response to the initial stimuli. Reporter gene assays couple the biological activity of a target to the expression of a readily detected enzyme or protein reporter, allowing monitoring of the cellular events associated with signal transduction and gene expression. Based upon the fusion of transcriptional control elements to a variety of reporter genes, these systems "report" the effects of a cascade of signaling events on gene expression inside cells. Synthetic repeats of a particular response element can be inserted upstream of the reporter gene to regulate its expression in response to signaling molecules generated by activation of a specific pathway in a live cell. Such assays have proven useful in primary and secondary screening of chemical libraries and drug leads for their biological effects and panels of transcription reporter assays have been used o profile drug activity (Bionaut, Inc.) Although transcription reporter assays have the capacity to provide information on the response of a pathway to chemical agents, such assays only measure the consequence of pathway activation or inhibition, and not the site of action of the compound.

Direct measures of specific events within signaling pathways would eliminate the problems associated with interpretation of transcriptional profiles. Unlike transcriptional reporter assays, the information obtained by monitoring a protein-protein interaction reflects the effect of a drug on a particular branch or node of a cell signaling pathway, not its endpoint. For example, stimulation of a pathway by an agonist could lead to an increase in the association of an intracellular protein (such as a kinase) with a cognate binding partner (such as a substrate). The stimulation results in the phosphorylation of the substrate by the kinase. The drug effect could therefore be assessed by quantifying the phosphorylation of the substrate or the amount or location of the kinase/substrate complex in the absence and presence of the agonist. In this example the kinase/substrate complex serves as a sentinel of pathway activity. A drug acting either at the beginning of the pathway (such as a receptor antagonist) or acting on another target downstream of the receptor could alter either the amount or location or modification status of the kinase/substrate complex within the cell. Thus, assessing the kinase/substrate complex in the absence or presence of a chemical compound would reveal whether or not the chemical compound acts on that pathway. Ideally such changes would be measured in intact cells. That is, cells of interest would be treated with the test compound for a defined period of time and the pathway sentinel or interaction would be assessed in the intact (live or fixed) cell.

High-content assays hold particular promise as they are capable of discriminating events that occur at the subcellular level. Pathways are often highly organized in subcellular space, with membrane receptors that receive signals at the plasma membrane and transmit those signals to the cell nucleus, resulting in the activation or repression of gene transcription. With high-content assays, fluorescence signals in the membrane, cytosol, nucleus and other subcellular compartments can be discriminated. Such assays can be performed by automated microscopy allowing throughput of hundreds of microwell plates per day, making the approach practical on a moderate-to large scale.

With the exception of our own work cited below and in the References herein (Michnick et al.) the prior art is silent on the use of protein-protein interactions for pharmacological profiling. In addition the prior art is silent on the use of high-content assays for pharmacological profiling and lead attrition.

Several methods are available for the quantification of protein-protein interactions. For the purposes of the present invention we focus on methods that enable the quantification and/or localization of protein-protein complexes in live cells while recognizing that additional methods, such as fluorescence polarization, are suitable for the measurement of binding events. Available cell-based methods involve fluorescent or bioluminescent assays of protein-protein interactions, and include resonance energy transfer (FRET or BRET), enzyme subunit complementation, and protein-fragment complementation assays (PCA). It will be understood by one skilled in the art that the present invention is not limited to the exact assay methodology that is selected, to the detection method, or to particular instrumentation. The present invention teaches that protein-protein interactions can be used for pharmacological profiling regardless of the particular technology or instrumentation applied as long as the technology is sufficiently sensitive and robust for assay purposes.

The most widespread fluorescent, cell-based protein-protein interaction assays are based on the phenomenon of fluorescence resonance energy transfer (FRET) or bioluminescence resonance energy transfer (BRET). In a FRET assay the genes for two different fluorescent reporters, capable of undergoing FRET are separately fused to genes encoding of interest, and the fusion proteins are co-expressed in live cells. When a protein complex forms between the proteins of interest, the fluorophores are brought into proximity if the two proteins possess overlapping emission and excitation, emission of photons by a first, "donor" fluorophore, results in the efficient absorption of the emitted photons by the second, "acceptor" fluorophore. The FRET pair fluoresces with a unique combination of excitation and emission wavelengths that can be distinguished from those of either fluorophore alone in living cells. As specific examples, a variety of GFP mutants have been used in FRET assays, including cyan, citrine, enhanced green and enhanced blue fluorescent proteins. With BRET, a luminescent protein- for example the enzyme Renilla luciferase (RLuc) -is used as a donor and a green fluorescent protein (GFP) is used as an acceptor molecule. Upon addition of a compound that serves as the substrate for Rluc, the FRET signal is measured by comparing the amount of blue light emitted by Rluc to the amount of green light emitted by GFP. The ratio of green to blue increases as the two proteins are brought into proximity. Newer methods are in development to enable deconvolution of FRET from bleedthrough and from autofluorescence. In addition, fluorescence lifetime imaging microscopy (FLIM) eliminates many of the artifacts associates with quantifying simple FRET intensity.

A variety of assays have been constructed based either on activity of wild-type beta-galactosidase or on the phenomenon of alpha- or omega-complementation. Beta-gal is a multimeric enzyme which forms tetramers and octomeric complexes of up to 1 million Daltons. beta-gal subunits undergo self-oligomerization which leads to activity. This naturally-occurring phenomenon has been used to develop a variety of in vitro, homogeneous assays that are the subject of over 30 patents. Alpha- or omega-complementation of beta-gal, which was first reported in 1965, has been utilized to develop assays for the detection of antibody-antigen, drug-protein, protein-protein, and other bio-molecular interactions. The background activity due to self-oligomerization has been overcome in part by the development of low-affinity, mutant subunits with a diminished or negligible ability to complement naturally, enabling various assays including for example the detection of ligand-dependent activation of the EGF receptor in live cells.

PCA represents a particularly useful method for measurements of the association, dissociation or localization of protein-protein complexes within the cell. PCA enables the determination and quantitation of the amount and subcellular location of protein-protein complexes in living cells. With PCA, proteins are expressed as fusions to engineered polypeptide fragments, where the polypeptide fragments themselves (a) are not fluorescent or luminescent moieties; (b) are not naturally-occurring; and (c) are generated by fragmentation of a reporter. Michnick et al. (US 6,270,964) taught that any reporter protein of interest can be used for PCA, including any of the reporters described above. Thus, reporters suitable for PCA include, but are not limited to, any of a number of enzymes and fluorescent, luminescent, or phosphorescent proteins. Small monomeric proteins are preferred for PCA, including monomeric enzymes and monomeric fluorescent proteins, resulting in small (~150 amino acid) fragments. Since any reporter protein can be fragmented using the principles established by Michnick et al., assays can be tailored to the particular demands of the cell type, target, signaling process, and instrumentation of choice. Finally, the ability to choose among a wide range of reporter fragments enables the construction of fluorescent, luminescent, phosphorescent, or otherwise detectable signals; and the choice of high-content or high-throughput assay formats. enabling various assays including for example the detection of ligand-dependent activation of the erythropoietin receptor in live cells.

Fluorescent assays of protein-protein interactions have been used individually to detect drug effects on individual targets, including receptors and other intracellular targets. For example, we have used PCA to construct cell-based, fluorescent, high-throughput screening assays based on the NFkappaB (p65/p50) complex (Yu et al.) However, the prior art is silent on the use of panels of such assays for pharmacological profiling.

In the process of making the present invention we tested three hypotheses. The first hypothesis was that high-content assays, and in particular quantitative, dynamic measurements of specific protein-protein complexes within specific pathways would enable an assessment of the activation or inhibition of those pathways by a chemical compound or agent. The second hypothesis was that two types of dynamic events could occur in response to pathway activation: an increase or decrease in the amount of a specific complex, and/or the translocation of a particular protein-protein complex from one subcellular compartment to another. The third hypothesis was that quantification and localization of a number and variety of distinct protein complexes within living cells would enable drug profiling on a global, cell-wide scale.

In making the present invention, cell-based assays were used to monitor the dynamic association and dissociation of proteins in the absence or presence of chemical compounds. Although any of the above-mentioned fluorescent or luminescent methods are suitable for use in conjunction with the present invention, we chose to use a protein-fragment complementation assay (PCA) strategy. We created panels of quantitative, fluorescent assays for distinct protein-protein interactions in live cells, and tested the activities of over 100 known drugs against the assay panels. We demonstrate that the pattern of responses or "pharmacological profiles" detected by changes in intensity and/or physical location of the sentinel pair is related to the mechanism of action, specificity, and off-pathway effects of the drug being tested.

### OBJECTS AND ADVANTAGES OF THE INVENTION

It is an object of the present invention to provide a method for pharmacological profiling of drugs, drug candidates, and drug leads on a broad scale.

It is a further object of the present invention to provide methods for assessing the activity, specificity, potency, time course, and mechanism of action of chemical compounds on a genome-wide scale.

It is also an object of the invention to allow determination of the selectivity of a chemical compound within the context of a living cell.

It is an additional object of the present invention to allow detection of the potential off-pathway effects, adverse effects, or toxic effects of a chemical compound within the biological context of a cell of interest.

It is an additional object of the invention to enable lead optimization, by performing pharmacological profiling of a collection or a series of lead compounds in an iterative manner until a desired pharmacological profile is obtained.

A further object of the invention is to enable attrition of drug candidates with undesirable or toxic effects.

It is a further object of the present invention to establish pre-clinical safety pro files for new drug candidates.

It is a further object of the present invention to improve the efficiency of the drug discovery process by identifying adverse, toxic or other off-pathway effects prior to clinical trials.

It is a further object of the present invention to improve the safety of first-in-class drugs by identifying adverse, toxic or other off-pathway effects prior to clinical trials.

It is a further object of the present invention to provide methods suitable for the development of 'designer drugs' with predetermined properties.

An additional object of the invention is to enable the identification of new therapeutic indications for known drugs.

Another object of this invention is to provide a method for analyzing the activity of any class of pharmacological agent on any biochemical pathway.

A further object of this invention is to enable the identification of the biochemical pathways underlying drug toxicity.

A further object of this invention is to enable the identification of the biochemical pathways underlying drug efficacy for a broad range of diseases.

A further object of this invention is to provide high-content methods, assays and compositions useful for drug discovery and evaluation.

An additional object of the invention is to provide panels of protein-protein interactions suitable for pharmacological profiling.

The present invention has the advantage of being broadly applicable to any pathway, gene, gene library, drug target class, reporter protein, detection mode, synthetic or natural product, chemical entity, assay format, automated instrumentation, or cell type of interest.

### SUMMARY OF THE INVENTION

The present invention seeks to fulfill the above-mentioned needs for pharmaceutical discovery. The present invention teaches that cell-based assays can be used to identify the mechanism of action, selectivity, and adverse or off-pathway effects of pharmacologically active agents. The present invention provides a general strategy for carrying out drug analysis and pharmacological profiling with cell-based assays, in particular, protein-protein interaction assays. In addition, the present invention provides a broad range of useful methods and compositions to fulfill these objectives.

Cellular responses are mediated by a complex network of proteins that are resident within subcellular compartments. Cell proliferation, cell-death (apoptosis), chemotaxis, metastases etc. are all controlled at the level of the proteins that participate in protein-protein interactions. The subject of this invention is a methodology that allows the quantitative analysis of the effects of chemical compounds on biochemical networks. The present invention enables an analysis of the effects of any chemical compound regardless of drug class or target class.

The novel methodology of this invention enables: (1) Direct visualization of the molecular architecture of specific cellular responses at the level of the discrete protein-protein interactions that enable such cellular architecture; (2) Direct and quantitative analysis of drug effects on cellular signaling networks in a manner never before possible; and (3) The creation of quantitative and predictive pharmacological profiles of lead compounds and drugs regardless of their mechanism of action.

A preferred embodiment of the invention uses gene(s) encoding specific proteins of interest; preferably as characterized full-length eDNA(s). The methodology is not limited, however, to full-length clones as partial cDNAs or protein domains can also be employed. The cDNAs, tagged with a reporter or reporter fragment allowing the measurement of a protein-protein interaction, are inserted into a suitable expression vector and the fusion proteins are expressed in a cell of interest. However, endogenous cellular genes can be used by tagging the genome with reporters or reporter fragments, for example by non-homologous recombination. In the latter case, the native proteins are expressed along with the reporter tags of choice enabling the detection of native protein-protein complexes.

The method requires a method for measuring protein-protein interactions and/or an equivalent, high-content assay method for a pathway sentinel. In a preferred embodiment, protein interactions are measured within a cell. Such methods may include, but are not limited to, FRET, BRET, two-hybrid or three-hybrid methods, enzyme subunit complementation, and protein-fragment complementation (PCA) methods. In alternative embodiments, the interactions are measured in tissue sections, cell lysates or cell extracts or biological extracts. In the latter cases, a wide variety of analytical methods for the measurement of protein-protein complexes can be employed, for example, immunohistochemistry; western blotting; immunoprecipitation followed by two-dimensional gel electrophoresis; mass spectroscopy; ligand binding; quantum dots or other probes; or other biochemical methods for quanitfying the specific protein-protein complexes. Such methods are well known to those skilled in the art. It should be emphasized that it is not necessary to apply a single technology to the measurement of the different protein-protein interactions. Any number or type of quantitative assays can be combined for use in conjunction with the present invention.

Yeast two-hybrid methods (Y2H) can be used with this invention although several features of Y2H make it less useful than direct fluorescent methods. First, Y2H often requires the expression of the proteins of interest within the nucleus of a cell such as the yeast cell, which is an unnatural context for most human proteins and cannot be used at all for human membrane proteins such as receptors. Second, yeast do not contain the human biochemical pathways that are of interest for drug discovery, which obviates pathway-based discovery and validation of novel, potential drug target proteins. Third, except for chemicals that directly disrupt protein-protein interactions, Y2H is not of use in identifying pharmacologically active molecules that disrupt mammalian biochemical pathways.

Enzyme-fragment complementation and protein-fragment complementation methods are preferred embodiments for this invention. These methods enable the quantification and subcellular localization of protein-protein complexes in living cells.

With enzyme fragment complementation, proteins are expressed as fusions to enzyme subunits, such as the naturally-occurring or mutant alpha/beta subunits of beta-galactosidase. With PCA, proteins are expressed as fusions to synthetic polypeptide fragments, where the polypeptide fragments themselves (a) are not fluorescent or luminescent moieties; (b) are not naturally-occurring; and (c) are generated by fragmentation of a reporter. Michnick et al. (US 6,270,964) taught that any reporter protein of interest can be used in PCA, including any of the reporters described above. Thus, reporters suitable for PCA include, but are not limited to, any of a number of enzymes and fluorescent, luminescent, or phosphorescent proteins. Small monomeric proteins are preferred for PCA, including monomeric enzymes and monomeric fluorescent proteins, resulting in small (~150 amino acid) fragments. Since any reporter protein can be fragmented using the principles established by Michnick et al., assays can be tailored to the particular demands of the cell type, target, signaling process, and instrumentation of choice. Finally, the ability to choose among a wide range of reporter fragments enables the construction of fluorescent, luminescent, phosphorescent, or otherwise detectable signals; and the choice of high-content or high-throughput assay formats.

As we have shown previously, polypeptide fragments engineered for PCA are not individually fluorescent or luminescent. This feature of PCA distinguishes it from other inventions that involve tagging proteins with fluorescent molecules or luminophores, such as US 6,518,021 (Thastrup et al.) in which proteins are tagged with GFP or other luminophores. A PCA fragment is not a luminophore and does not enable monitoring of the redistribution of an individual protein. In contrast, what is measured with PCA is the formation of a complex between two proteins.

The present invention is not limited to the type of cell, biological fluid or extract chosen for the analysis. The cell type can be a mammalian cell, a human cell, bacteria, yeast, plant, fungus, or any other cell type of interest. The cell can also be a cell line, or a primary cell, such as a hepatocyte. The cell can be a component of an intact tissue or animal, or in the whole body, such as in an explant or xenograft; or can be isolated from a biological fluid or organ. For example, the present invention can be used in bacteria to identify antibacterial agents that block key pathways; in fungal cells to identify antifungal agents that block key pathways. The present invention can be used in mammalian or human cells to identify agents that block disease-related pathways and do not have off-pathway or adverse effects. The present invention can be used in conjunction with drug discovery for any disease of interest including cancer, diabetes, cardiovascular disease, inflammation, neurodegenerative diseases, and other chronic or acute diseases afflicting mankind.

The present invention can be used in live cells or tissues in any milieu, context or system. This includes cells in culture, organs in culture, and in live organisms. For example, this invention can be used in model organisms such as Drosophila or zebrafish. This invention can also be used in nude mice, for example, human cells expressing labeled proteins ― such as with "PCA inside"- can be implanted as xenografts in nude mice, and a drug or other compound administered to the mouse. Cells can then be re-extracted from the implant or the entire mouse can be imaged using live animal imaging systems such as those provided by Xenogen (Alameda, California). In addition, this invention can be used in transgenic animals in which the protein fusions representing the protein-protein interactions to be analyzed are resident in the genome of the transgenic animal.

Any type of chemical compound can be analyzed with the methods provided herein. Such compounds include synthetic molecules, natural products, combinatorial libraries, known or putative drugs, ligands, antibodies, peptides, small interfering RNAs (siRNAs), or any other chemical agent whose activity is desired to be tested. Screening hits from combinatorial library screening or other high-throughput screening campaigns can be used in conjunction with the present invention. The invention can be used to identify those compounds with more desirable properties as compared with those compounds with less desirable properties. Therefore the present invention is suitable for use in optimization and/or attrition of lead compounds with unexpected, undesirable, or toxic attributes.

Although the preferred embodiment is a high-content assay format, high-throughput assays may be used in many if not all cases. In the case of an increase or decrease in the amount of a protein-protein complex in response to a chemical agent, the bulk fluorescent or luminescent signal can be quantified. In the event of a shift in the subcellular location of a protein-protein complex in response to drug, individual cells are imaged and the signal emanating from the protein-protein complex, and its sub-cellular location, is detected. Multiple examples of these events are provided herein. Some methods and reporters will be better suited to different situations. With PCA, a choice of reporters enables the quantification and localization of protein-protein complexes. Particular reporters may be more or less optimal for different cell types and for different protein-protein complexes.

It will be appreciate by one skilled in the art that, in many cases, the amount of a protein-protein complex will increase or decrease as a consequence of an increase or decrease in the amounts of the individual proteins in the complex. Similarly, the subcellular location of a protein-protein complex may change as a consequence of a shift in the subcellular location of the individual proteins in the complex. In such cases, either the complex or the individual components of the complex can be assessed and the results will be equivalent. High-content assays for individual pathway sentinels (proteins) can be constructed by tagging the proteins with a fluorophore or luminophore, such as with a green fluorescent protein (GFP) that is operably linked to the protein of interest; or by newer, self-tagging methods including SNAP tags and Halo-tags (Invitrogen, BioRad); or by applying immunofluorescence methods, which are well known to those skilled in the art of cell biology, using protein-specific or modification-specific antibodies provided by Cell Signaling Technologies, Becton Dickinson, and many other suppliers. Such methods and reagents can be used in conjunction with the protein-protein interactions provided herein. In particular, individual proteins - including those listed in Tables 1-2 - may be used to construct high-content assays for pharmacological profiling according to this invention.

The present invention also provides strategies and methods for detecting the effects of test compounds on modulable pathways in cells. The pathway modulation strategy can be applied to pharmacological profiling in conjunction with any cell type and with any measurable parameter or assay format. Whereas test compounds may not have significant effect under basal conditions, their effects can be detected by treating a cell with the test compound and then with a pathway modulator. This strategy improves the sensitivity of the invention. For example, in some cases a test compound may have no effect under basal conditions but may have a pronounced effect under conditions where a pathway is either activated or suppressed. Examples of the pathway modulation strategy are shown herein for GPCR pathways (+isoproterenol), cytokine pathways (+TNF) and DNA damage response pathways (+camptothecin). Any number of cellular pathways can be activated or suppressed by known modulators, which can be used to improve the sensitivity of pharmacological profiling.

The methods and assays provided herein may be performed in multiwell formats, in microtiter plates, in multispot formats, or in arrays, allowing flexibility in assay formatting and miniaturization. The choices of assay formats and detection modes are determined by the biology of the process and the functions of the proteins within the complex being analyzed. It should be noted that in either case the compositions that are the subject of the present invention can be read with any instrument that is suitable for detection of the signal that is generated by the chosen reporter. Luminescent, fluorescent or bioluminescent signals are easily detected and quantified with any one of a variety of automated and/or high-throughput instrumentation systems including fluorescence multi-well plate readers, fluorescence activated cell sorters (FACS) and automated cell-based imaging systems that provide spatial resolution of the signal. A variety of instrumentation systems have been developed to automate HCS including the automated fluorescence imaging and automated microscopy systems developed by Cellomics , Amersham, TTP , Q3DM (Beckman Coulter), Evotec, Universal Imaging (Molecular Devices) and Zeiss. Fluorescence recovery after photobleaching (FRAP) and time lapse fluorescence microscopy have also been used to study protein mobility in living cells. The pres ent invention can also be used in conjunction with the methods described in US 5,989,835 and US 6,544,790.

### BRIEF DESCRIPTION OF THE DRAWINGS

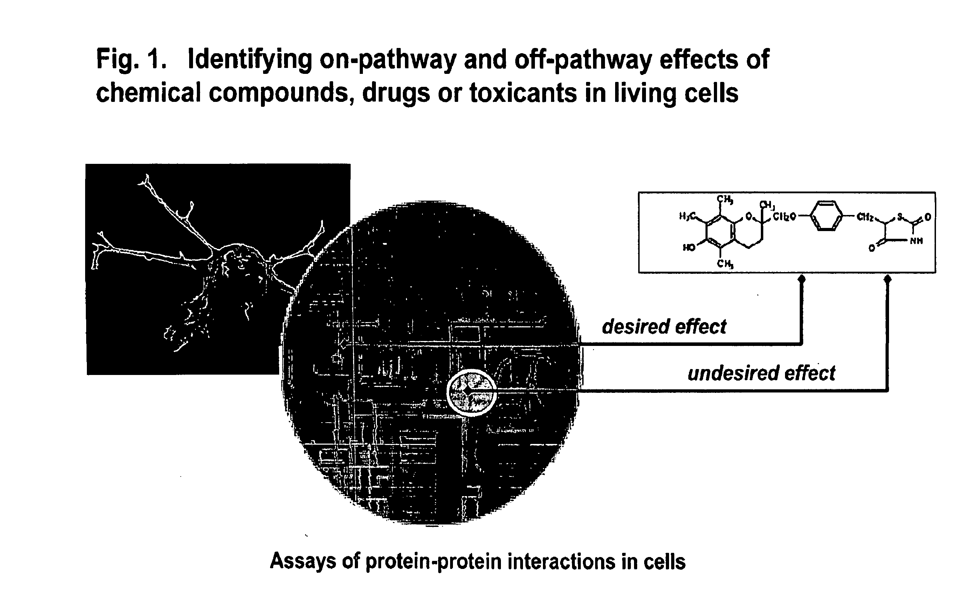

Figure 1 illustrates the objective of the present invention. The biochemical networks that control cellular behavior are represented as a circuit diagram. Drugs and chemical compounds have both known (intended) and unknown (unintended) effects within cells. Protein-protein interactions, or complexes, represent binary elements within biochemical networks that can be probed to identify known unknown effects of drugs and lead compounds.

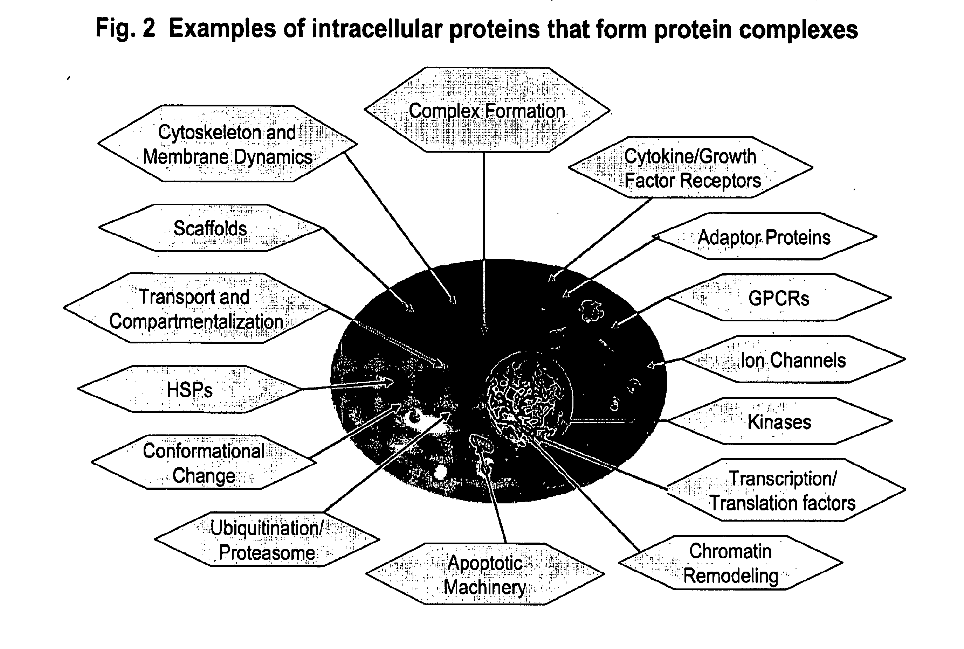

Figure 2 provides examples of intracellular proteins forming protein-protein complexes. Virtually every protein in the cell forms complexes with one or more other proteins or other macromolecules, and exerts its activity in the context of these macromolecular complexes. Examples of proteins forming complexes ― for which assays can be constructed ― include receptors (membrane, cytosolic or nuclear), ion channels, adaptor proteins, kinases, phosphatases, proteases, nucleotide binding proteins and nucleotide exchange factors, biosynthetic or catabolic enzymes, chaperones, exchange factors and transport proteins, the apoptotic, cytoskeletal and cell cycle machinery, scaffolds and structural proteins, the cytoskeleton and other structural elements, and transcription factors.

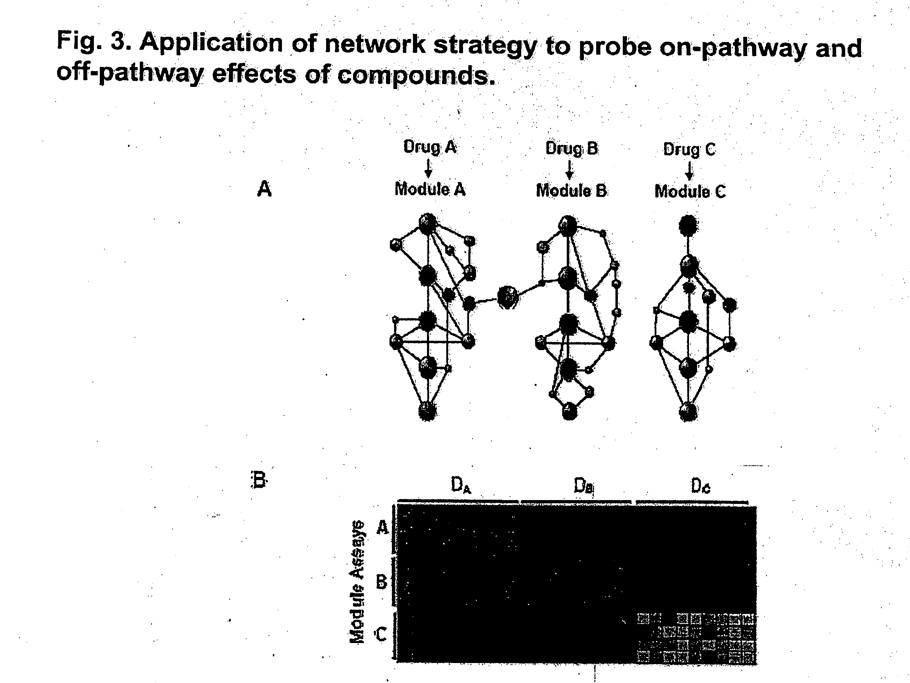

Figure 3 shows the concept relating intracellular networks to drug effects and to pharmacological profiles. (A) Biochemical signaling networks are comprised of modules or groups of functionally related protein-protein interactions. Three theoretical modules are represented as ball and stick diagrams (Modules A, B and C). Balls represent protein interactions that are connected within modules by lines. Signal transmission between Module A and Module B occurs via the complex shaded in blue. Each protein in a complex constitutes a potential assay to monitor upstream drug activity. Red and green shadings represent assays chosen to probe the response of the module to drugs A-C. Protein complexes that decrease in response to a particular drug treatment are shaded in red and those that increase in response to a particular drug treatment are shaded in green. (B) A matrix depicting the resulting quantitative patterns of inhibition (relative shades of red) and activation (relative shades of green) for various drugs (on the x axis) vs. individual assays (on the y axis). Drugs in category A (D_{A}) have an inhibitory effect on module A which is transmitted to module B. Thus the 4 assays representing module A, as well as those representing module B, decreased in activity. Drugs in category B (D_{B}) have an inhibitory effect on module B assays only, while drugs in category C (D_{C}) have a stimulatory effect on module C assays only.

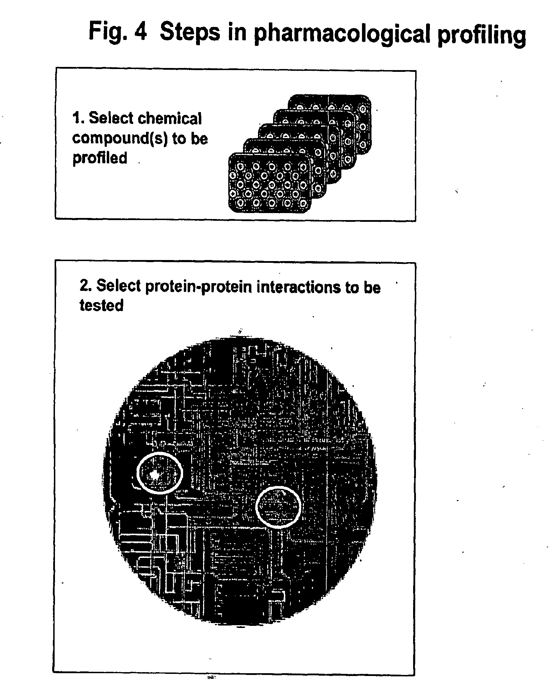

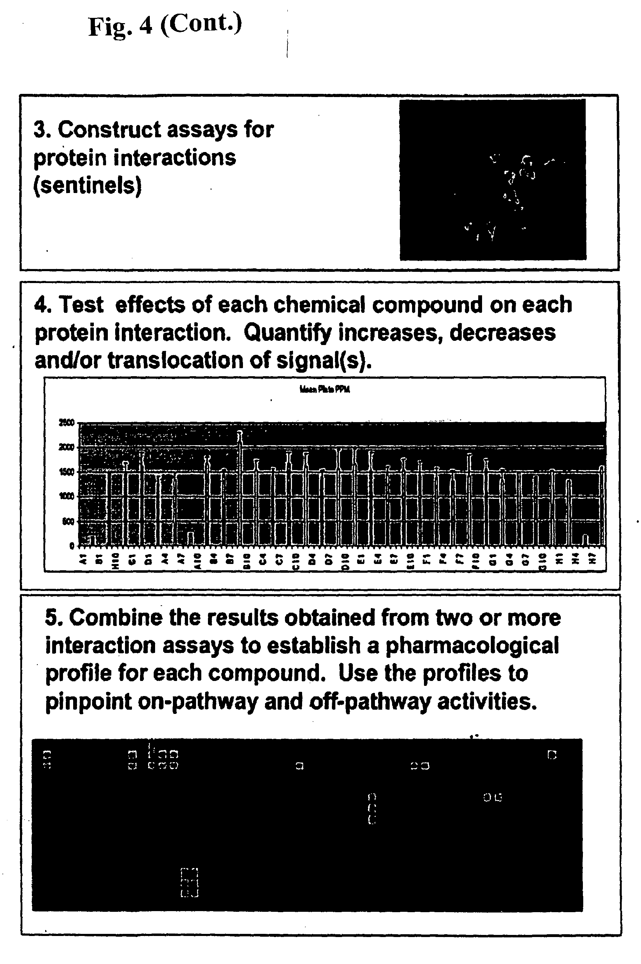

Figure 4 depicts the basic steps in pharmacological profiling according to the present invention.

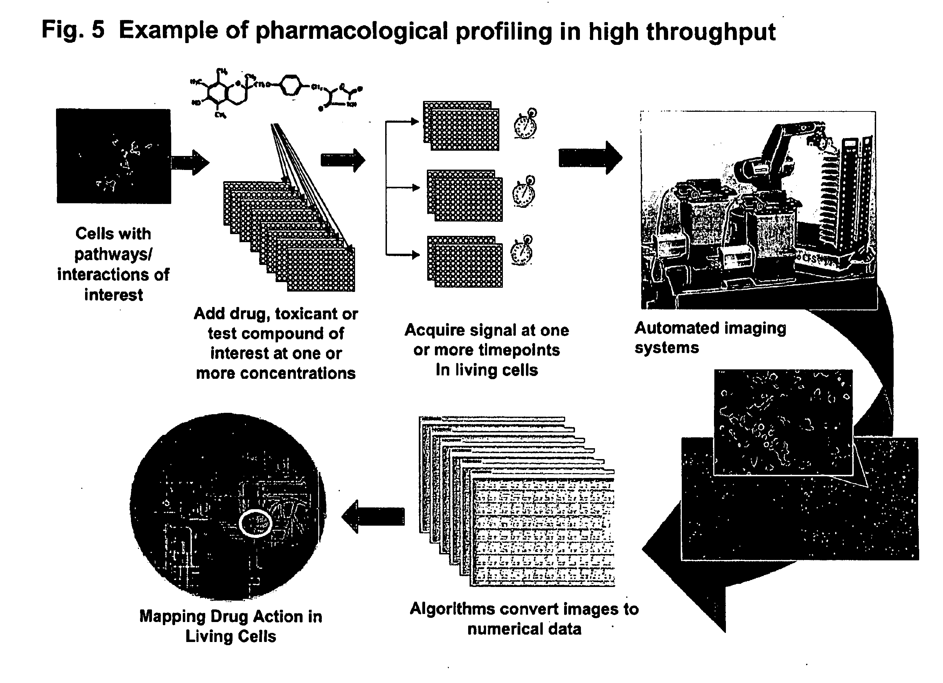

Figure 5 shows a high-throughput system for pharmacological profiling according to the present invention. The entire process can be automated in microtiter plate formats or arrays. Drugs can be analyzed at different time points after addition to cells, in order to obtain study the dynamics of drug action. The images are converted to numereical results and the numerical data can be stored in a relational database and analyzed by any number of graphical, numerical or statistical routines.

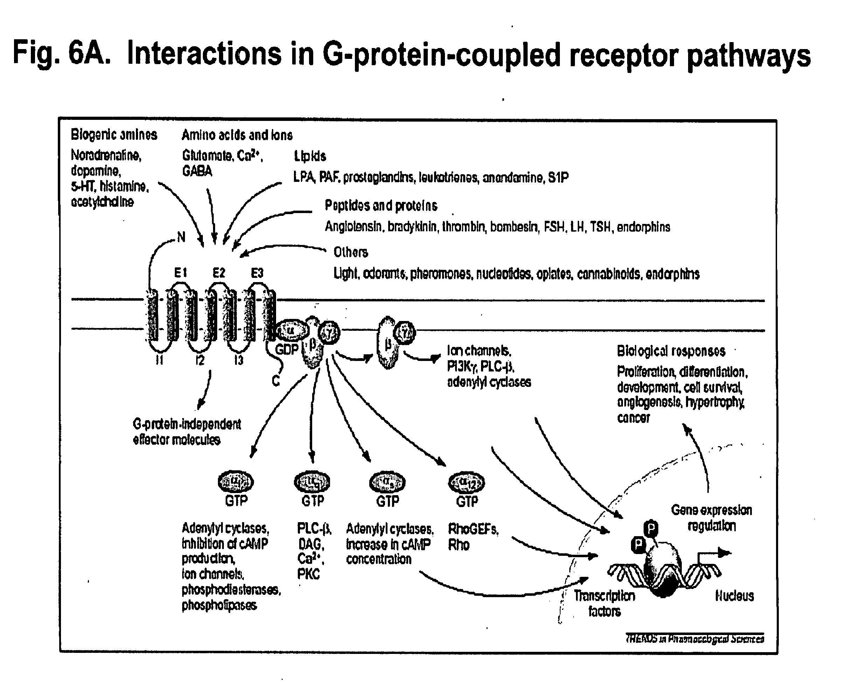

Figure 6A is a published schematic of GPCR pathways. G-protein-coupled receptors and their downstream interactors form protein-protein complexes that can be probed in intact cells.

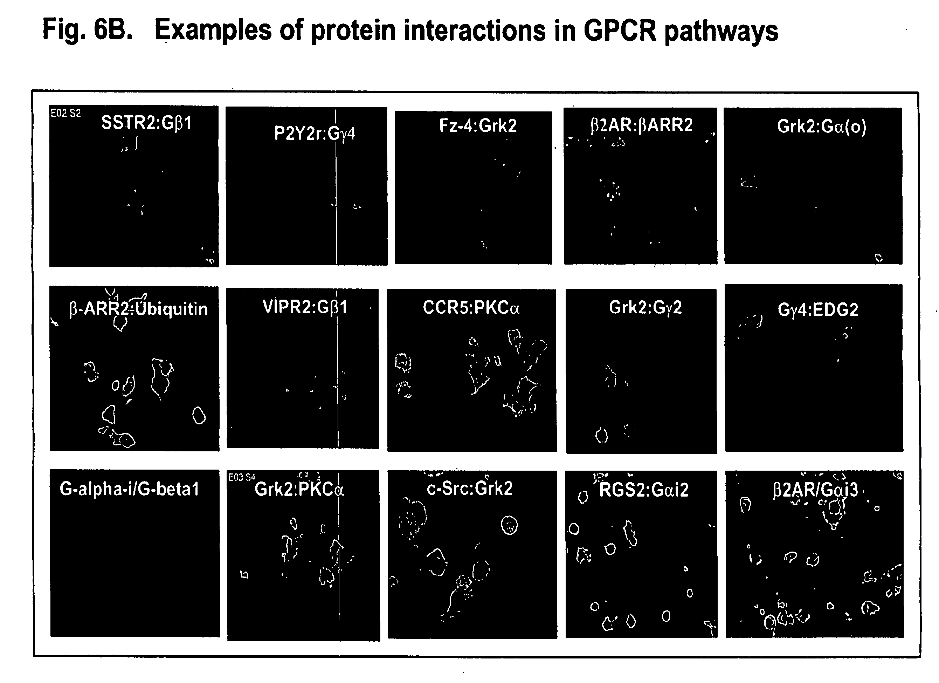

Figure 6B shows examples of protein complexes in GPCR pathways in intact human cells as assessed by PCA. The fluorescence signal reflects the amount and subcellular location of each protein-protein complex (green); nuclei were stained with Hoechst (blue). Refer to Tables 1-3 and the experimental protocol for details of the assays shown here. Such assays can be used in pharmacological profiling according to this invention.

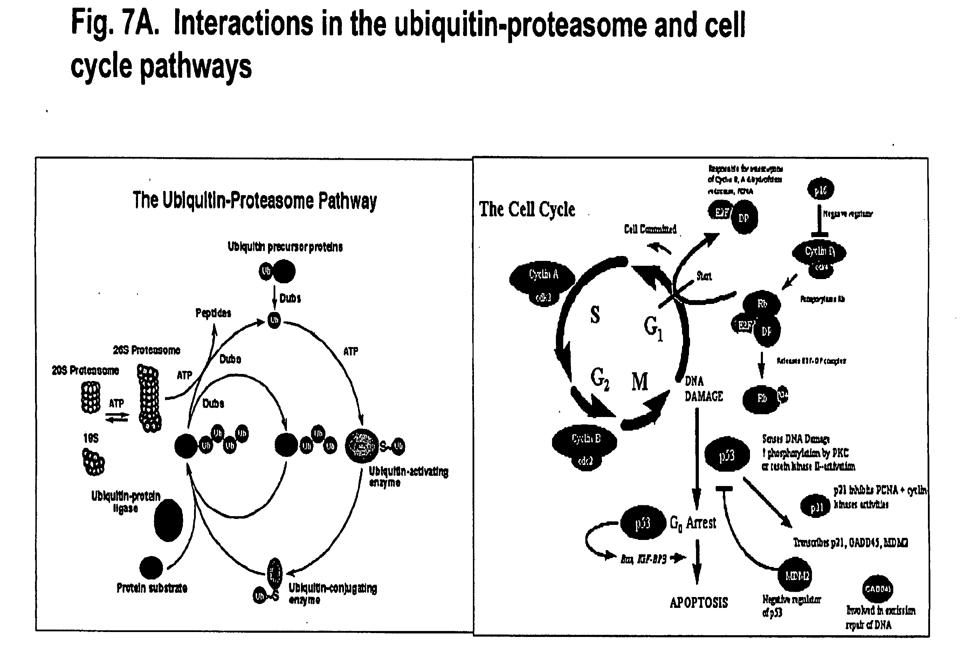

Figure 7A is a published schematic of interactions in the ubiquitin-proteasome and cell cycle pathways. Such pathways can be probed with protein-protein interactions.

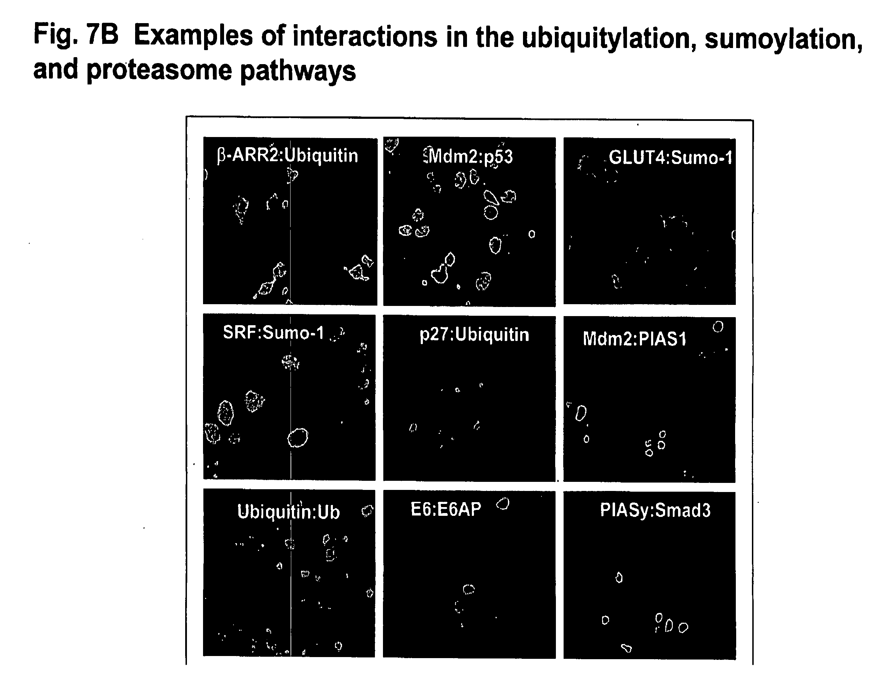

Figure 7B shows examples of protein complexes in the ubiquitylation, sumoylation and/or proteasome pathway. Direct interactions of proteins with ubiquitin or SUMO are shown, as are interactions of E3 and SUMO ligases with their substrates as assessed by PCA. Such assays can be used to detect the effects of chemical compounds on these pathways and can be constructed for many other proteins.

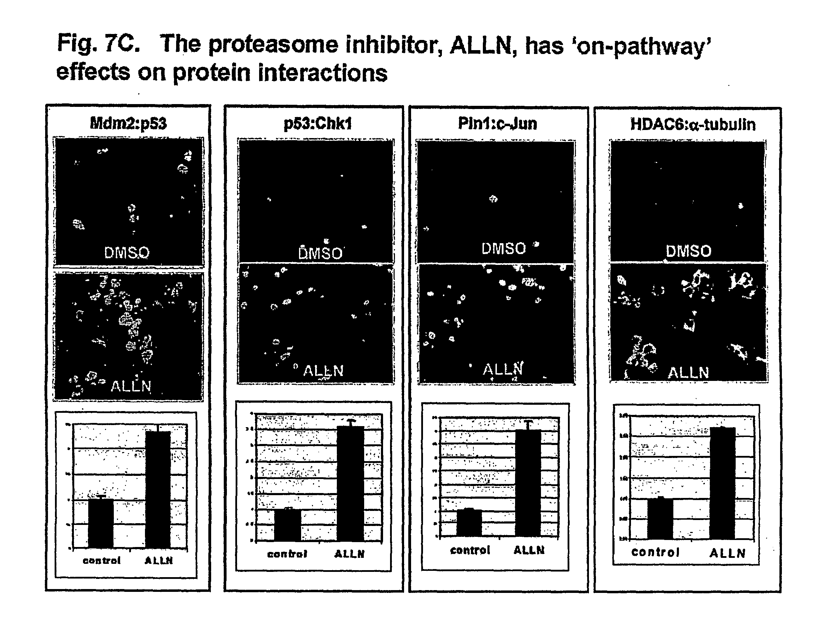

Figure 7C shows that the proteasome inhibitor, ALLN, causes the accumulation of protein-protein complexes that are known to be controlled by the proteasome; this effect of ALLN is therefore an expected or 'on-pathway' effect of the agent. Agents that inhibit or activate or potentiate these pathways can be identified with these assays.

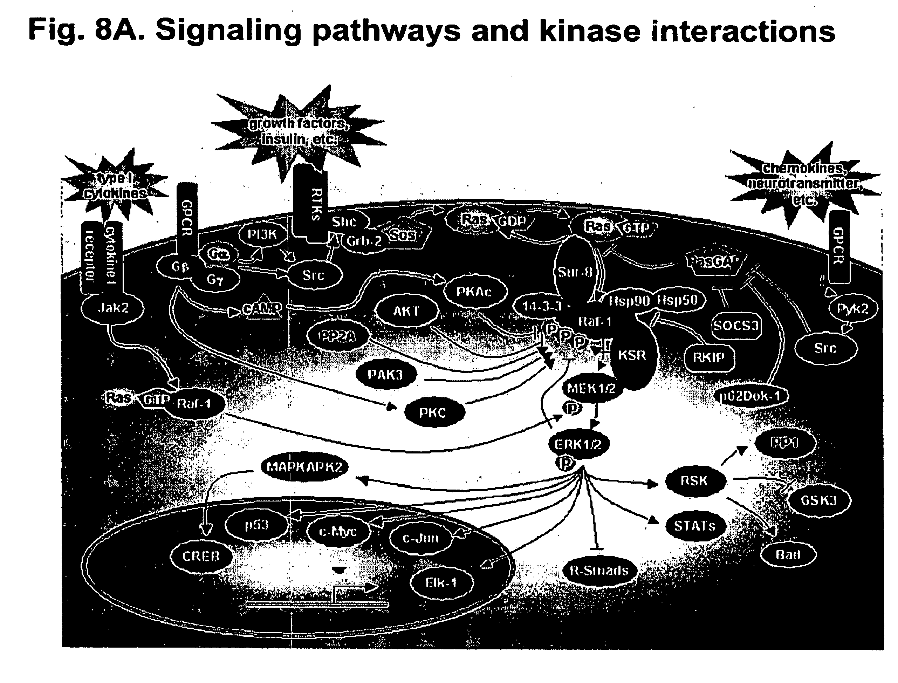

Figure 8A is a published schematic of interactions in signaling pathways, including interactions of various kinases with other proteins.

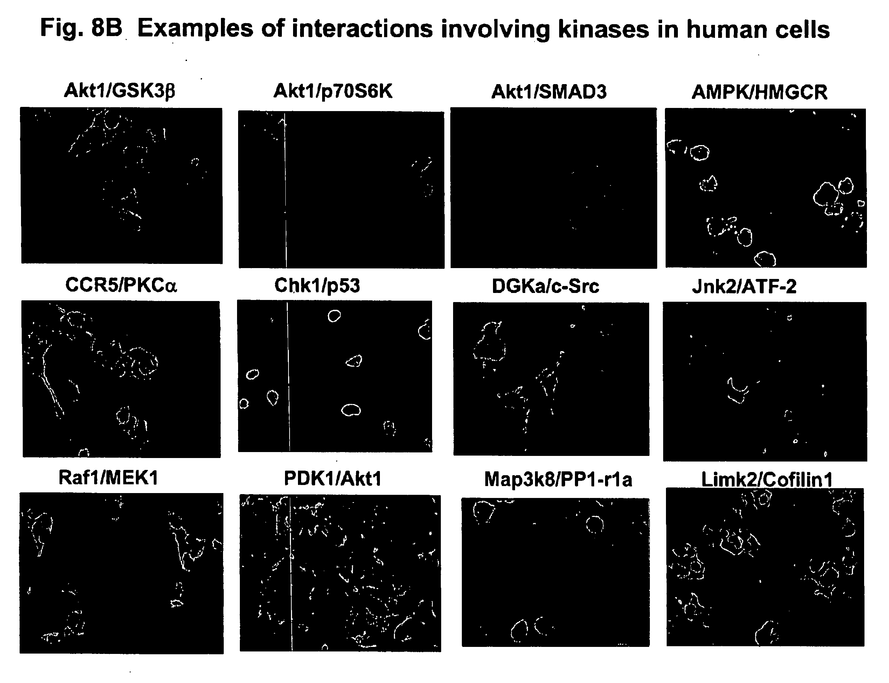

Figure 8B shows examples of interactions between kinases and other proteins in intact cells as assessed by PCA. Such assays can be constructed for many other kinases and signaling proteins, and can be used to detect the effects of known or novel chemical compounds on these pathways.

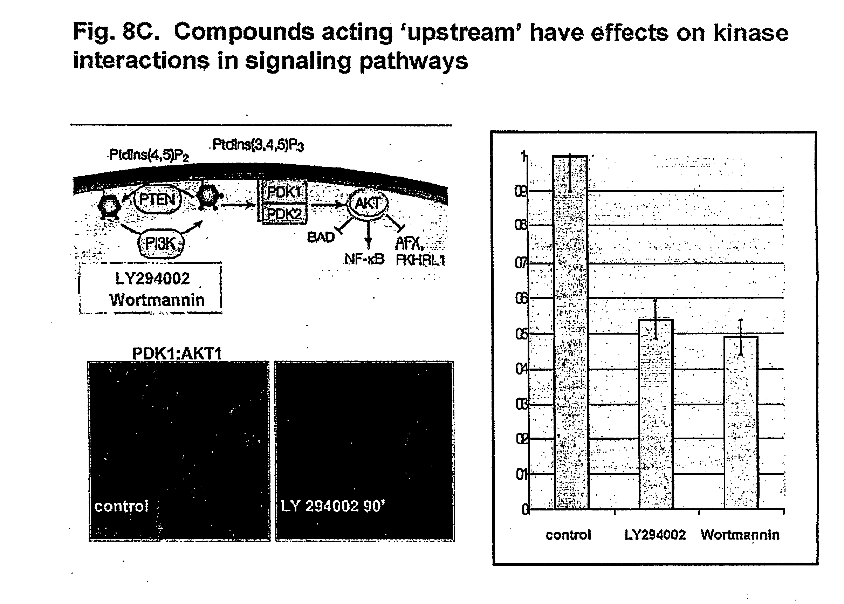

Figure 8C shows expected effects of 'upstream' kinase inhibitors on 'downstream' complexes. Wortmannin and Ly294002 are inhibitors of PI3K (phosphatidylinositol-3-kinase) which is upstream of PDK1 and AKT1. AKT1 is a substrate of PDK1 and forms a complex with PDK1. Cell treatment with PI3K inhibitors results in a loss of PDK1/AKT1 at the cell membrane and a relocalization of the complex to the cytosol, as shown in the images. Changes occur within minutes of drug addition; images were taken at 90 minutes. The terms 'upstream' and 'downstream' are commonly used to refer to signaling cascades, where events occur in a temporal sequence, with an 'upstream' event preceding a 'downstream' event.

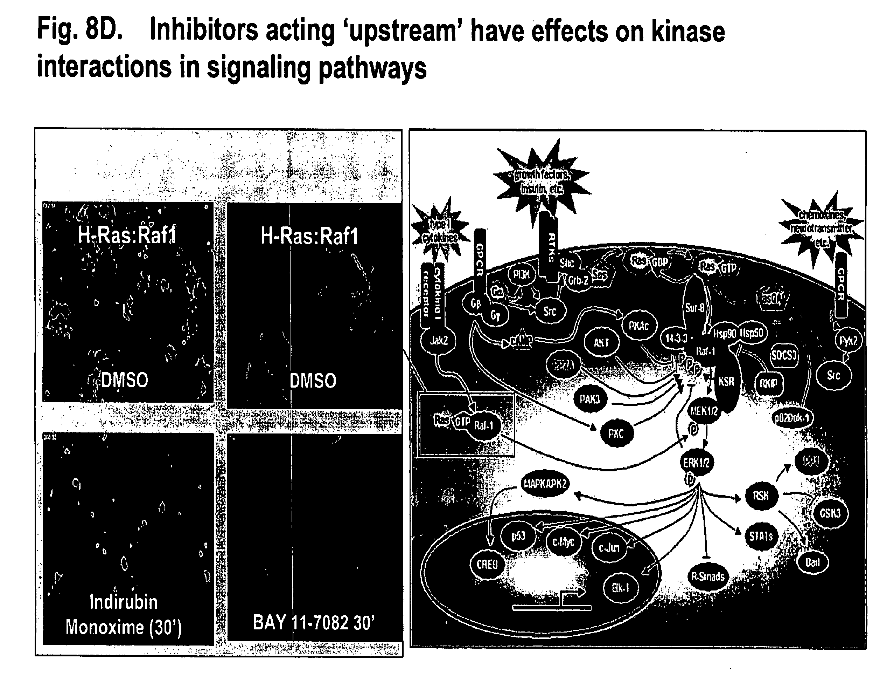

Figure 8D shows the effects of two different nonselective kinase inhibitors, indirubin-3'-monoxime and BAY11-7082, on Ras/Raf-1 complexes in HEK293 cells. The number of complexes decreases within minutes of drug treatment; images were taken at 30 minutes.

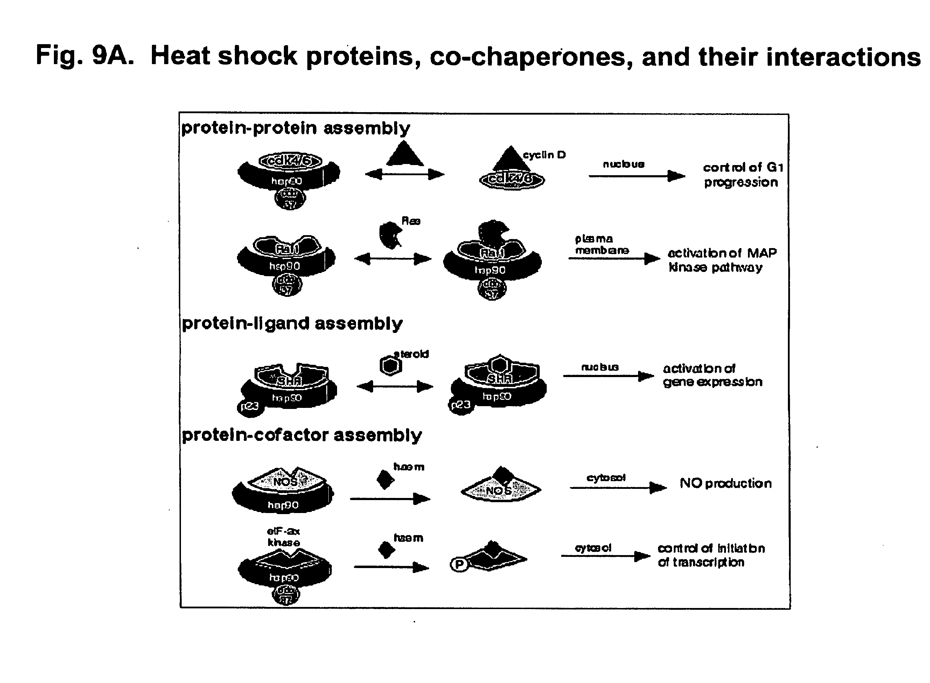

Figure 9A is a published schematic of selected interactions of chaperones and co-chaperones with each other and with client proteins.

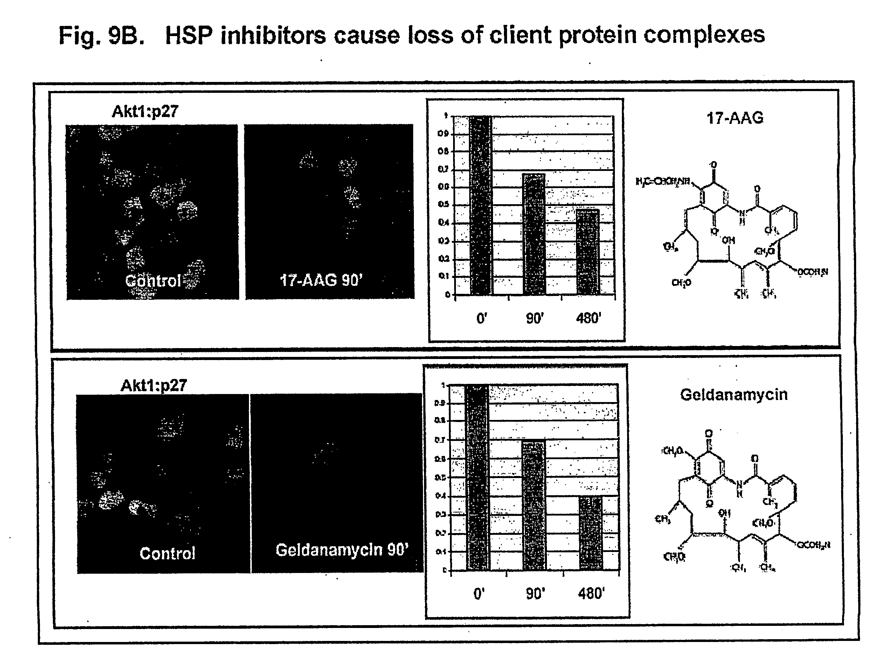

Figure 9B shows that treatment of cells with the known HSP inhibitors, geldanamycin or 17-allylamino-geldanamycin (17-AAG) causes a loss of client-protein complexes. Aktl is a client protein for HSP90. Effects are shown for Aktl/p27. Such assays can be used to assess the effects of known or novel compounds on these targets and pathways.

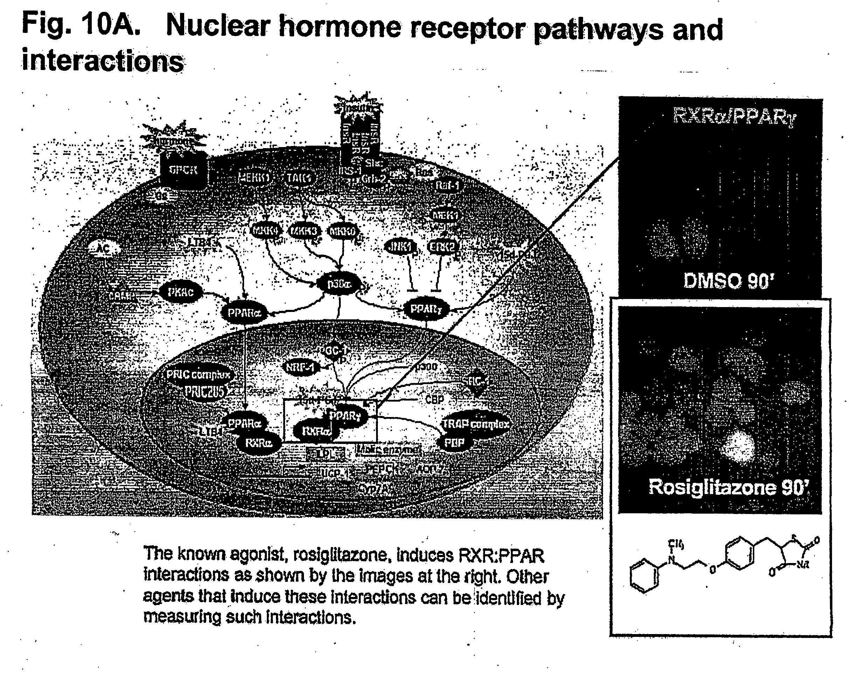

Figure 10A is a published schematic of interactions involving nuclear hormone receptors. The agonist, rosiglitazone, induces the formation of complexes between its receptor-PPARgamma- and a nuclear coactivator, in this case, RXRalpha.

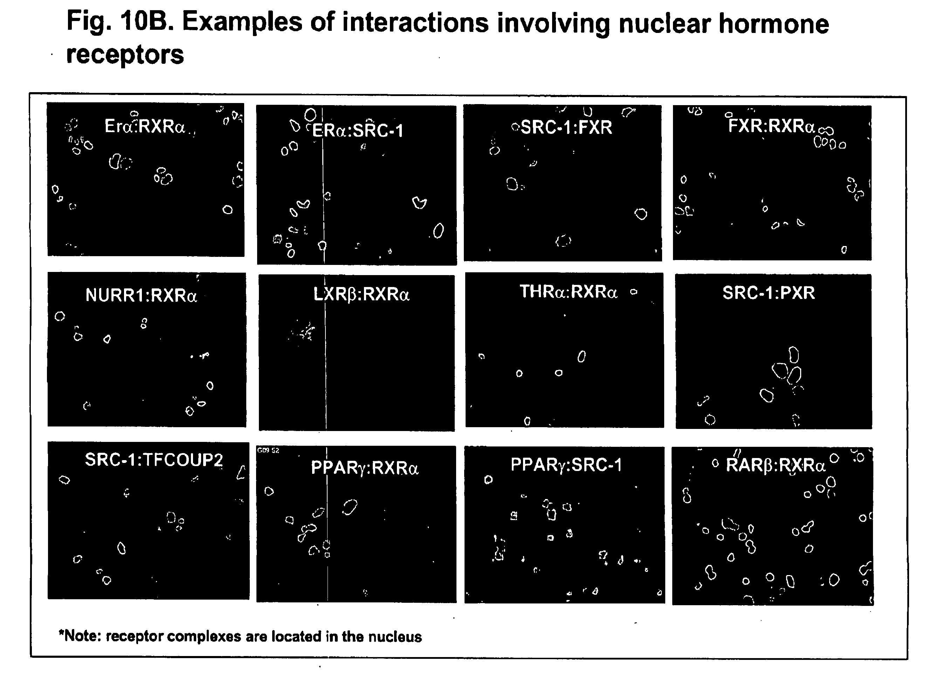

Figure 10B shows examples of several interactions involving nuclear hormone receptors. As in the other examples shown herein, assays were constructed using PCA. Such protein-protein interactions can be used to assess the effects of known or novel compounds on these pathways.

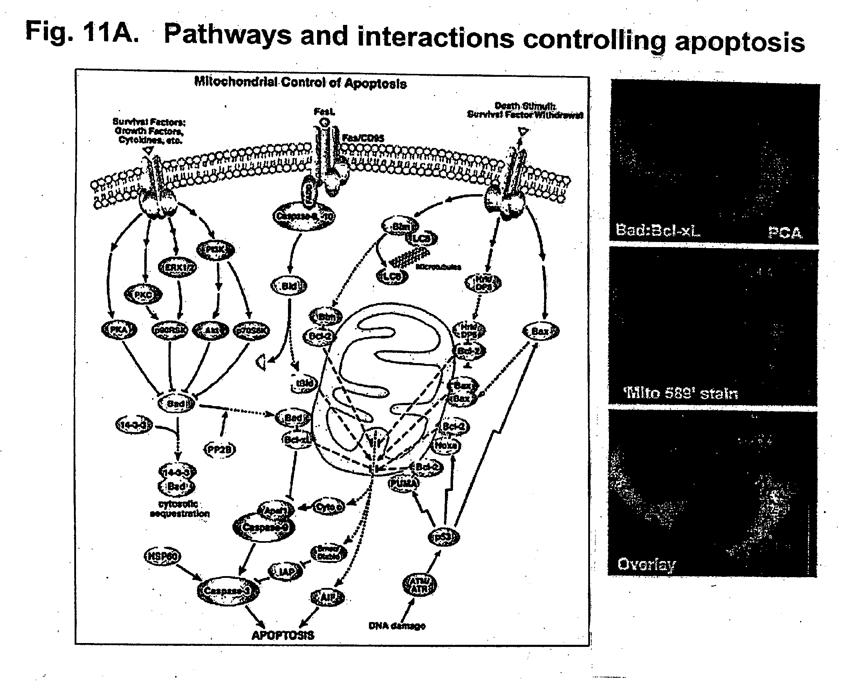

Figure 11A shows a published schematic of interactions in the mitochondrial pathway of apoptosis. The photomicrographs show the actual interaction of Bad/Bcl-xL in the mitochondria; the PCA signal (green) co-localizes with a mitochondrial stain (red) demonstrating that the protein complexes are correctly localized in the mitochondria.

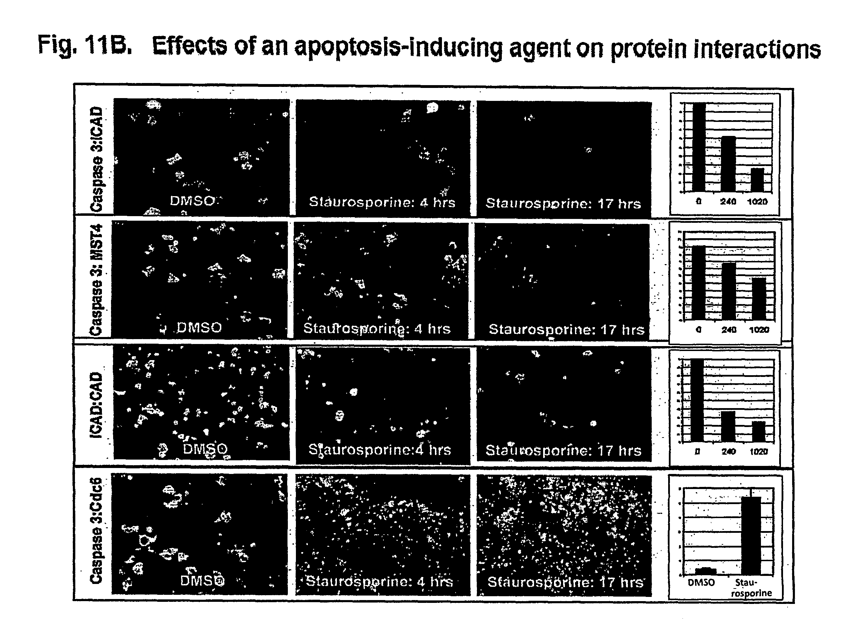

Figure 11B shows the dynamic effects of staurosporine, an apoptosis-inducing agent, on four different protein interactions. Fluorescence micrographs show the location and intensity of the complexes, and the histograms show quantitation of the signal based on quantitative image analysis. Such protein-protein interactions can be used to assess the effects of known or novel compounds on these pathways.

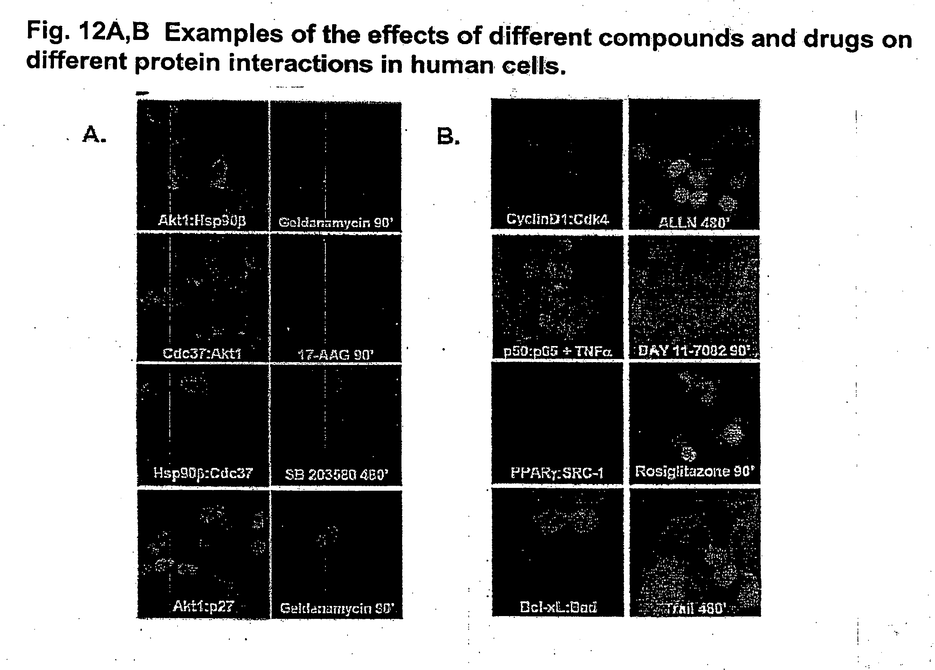

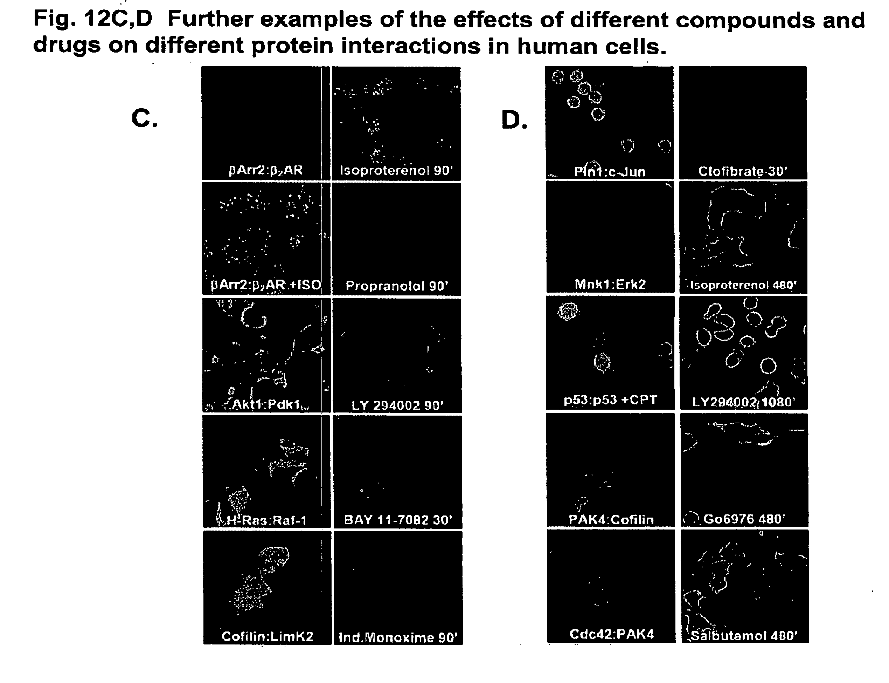

Figure 12 (A-D) shows images for different protein-protein interactions with representative drug effects. Details of the proteins and drugs used are in Table 1 and Table 4, respectively. Hoechst (blue) and YFP (green) fluorescence images are shown (40X objective). For each column, the left hand panel shows the control (vehicle only) and names the complex in white text, and the right hand panel shows the drug/time effect. Prior to assay termination, the assays labeled "+CPT" were stimulated with 500nM camptothecin for 960 minutes, the assay labelled '+ISO' was stimulated with 2 micromolar isoproterenol for 30 minutes, and the assay labelled '+TNFalpha' was stimulated with 50 ng/ml human TNFalpha for 20 minutes. The p50:p65 assay is shown without the nuclear Hoechst images to enable visualization of drug effects on the subcellular localization of the complex. The use of camptothecin, isoproterenol and TNFalpha to induce particular pathways is an example of the pathway modulation strategy provided in this invention.

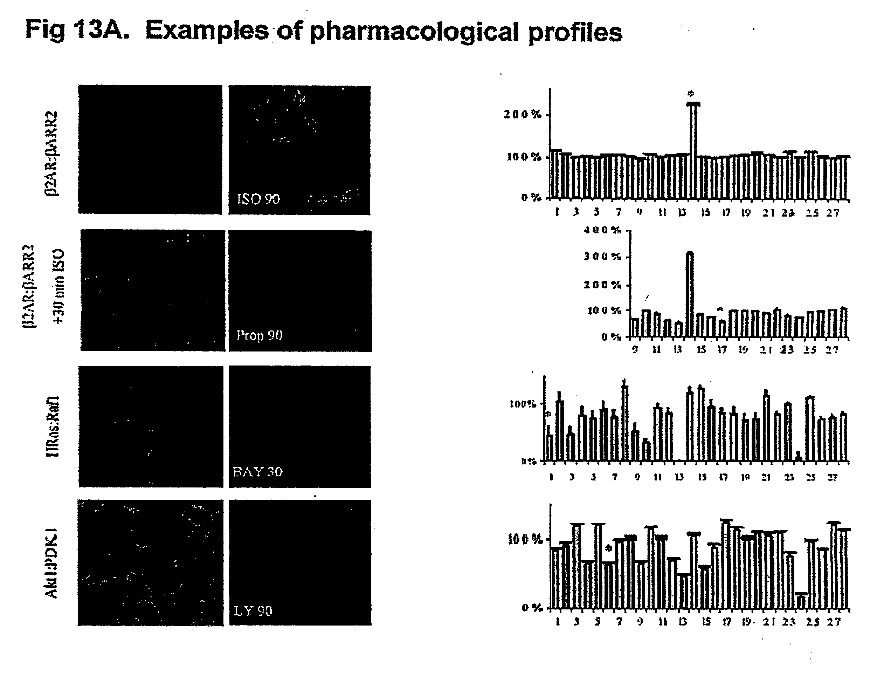

Figure 13A shows examples of quantitative drug effects on protein-protein interactions. 17 different drugs were tested against four distinct protein-protein interactions at multiple time points. Representative photomicrographs, showing subcellular localization of fluorescent protein-protein complexes, are shown on the left. Histograms reveal the quantitative results of the drug effects seen in the images which are marked with asterisks.

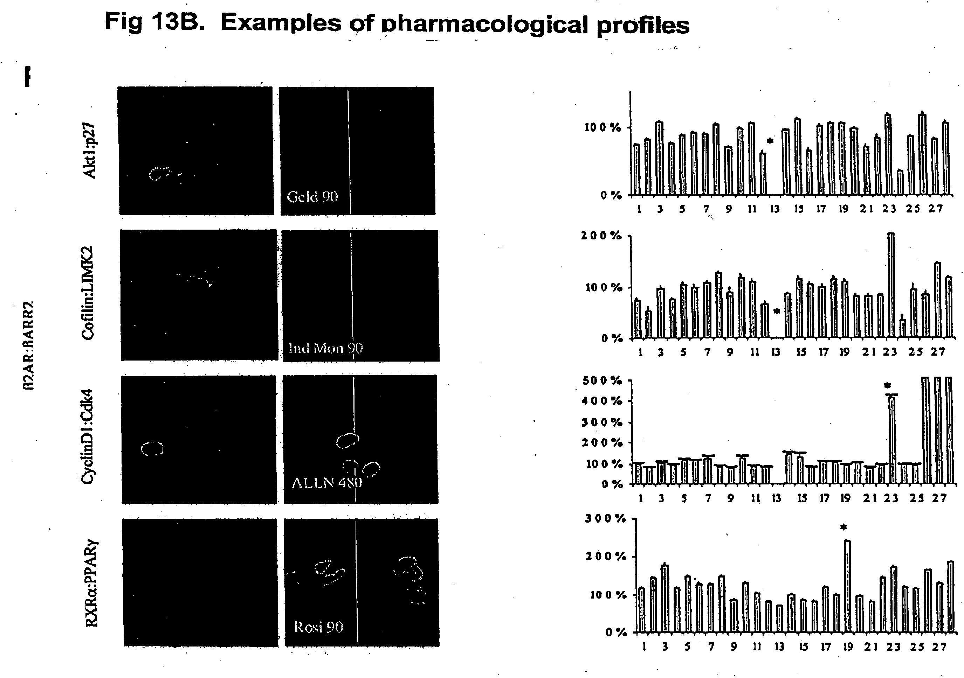

Figure 13B shows examples of drug effects on protein-protein interactions. 17 different drugs were tested against four distinct protein-protein interactions at multiple time points. Representative photomicrographs, showing subcellular localization of fluorescent protein-protein complexes, are shown on the left. Histograms show the quantitative results of the drug effects (asterisked).

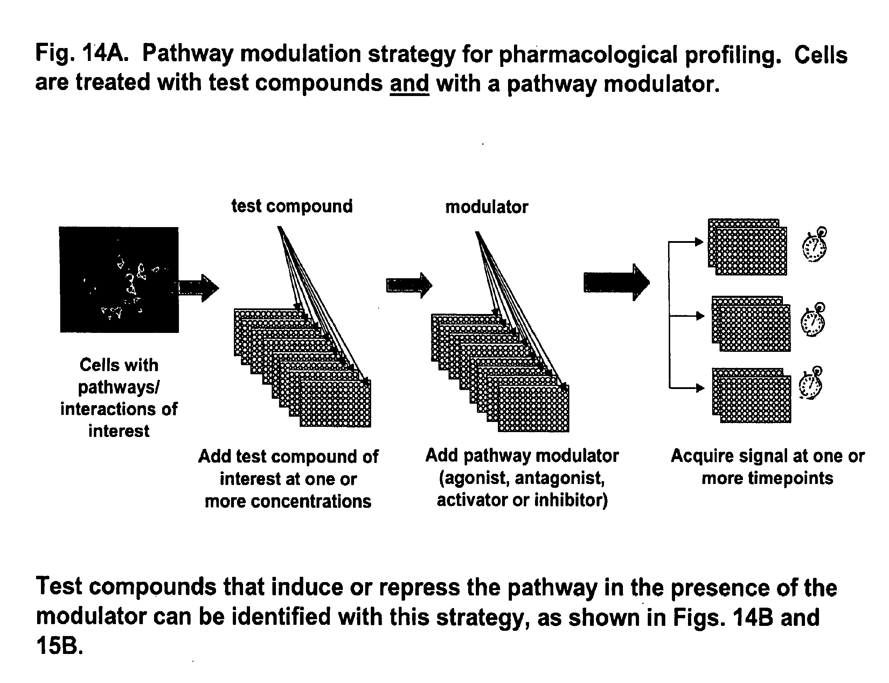

Figure 14A illustrates the pathway modulation strategy for pharmacological profiling. Cells are treated with a test compound followed by a pathway modulator of any chemical composition or type. With this strategy, test compounds that potentiate or block the effects of the modulator on cell pathways can be detected.

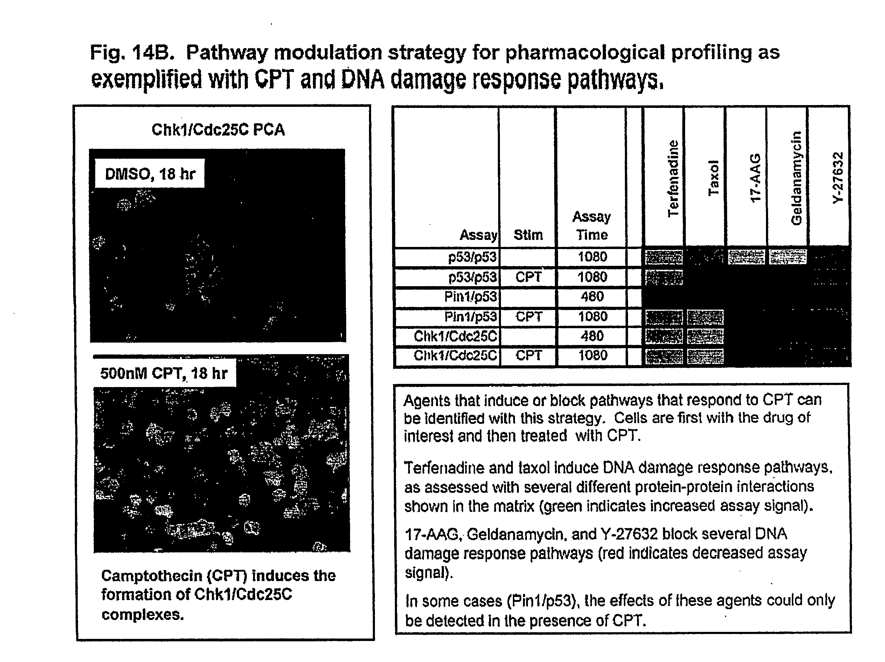

Figure 14B shows the utility of the pathway modulation strategy for pharmacological profiling. Camptothecin was used to induce DNA damage, leading to activation of DNA damage response pathways, as shown for Chkl/Cdc25C. Agents that potentiate or block the effects of camptothecin can then be identified, as shown in the matrix. A similar assay strategy can be taken with any cellular pathway, and with any pathway modulator.

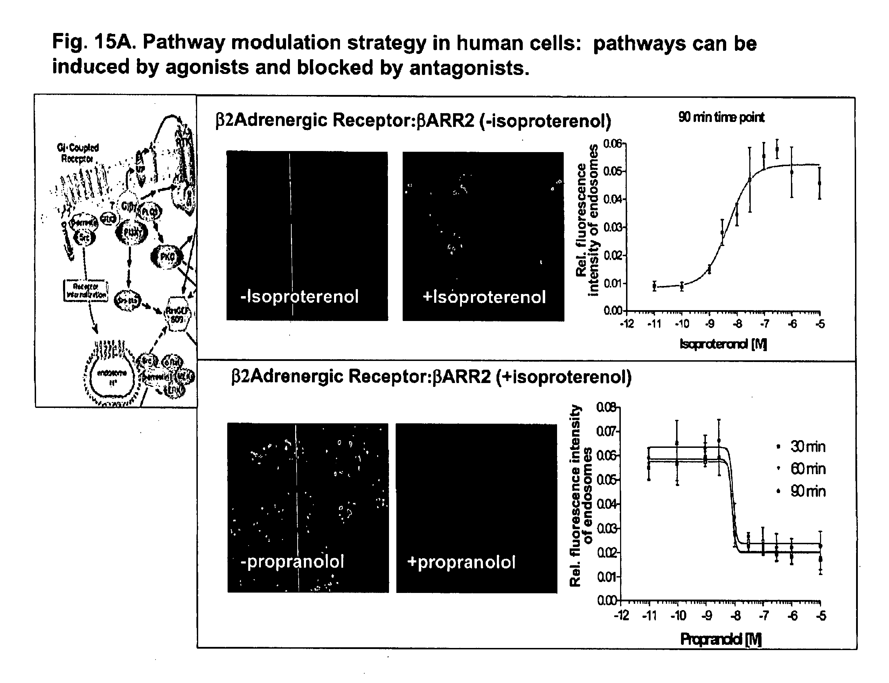

Figure 15A shows activation of a GPCR pathway by its known agonist and inhibition by antagonist. Isoproterenol induced the formation of complexes between the beta-adrenergic receptor (a GPCR) and beta-arrestin. Pretreatment with propanolol blocked the effect of isoproterenol. Thus, in the absence of isoproterenol, pathway activators can be detected; whereas in the presence of isoproterenol, pathway antagonists or inhibitors can be detected. A similar strategy can be applied to any cell receptor including GPCRs, membrane receptors, and nuclear receptors.

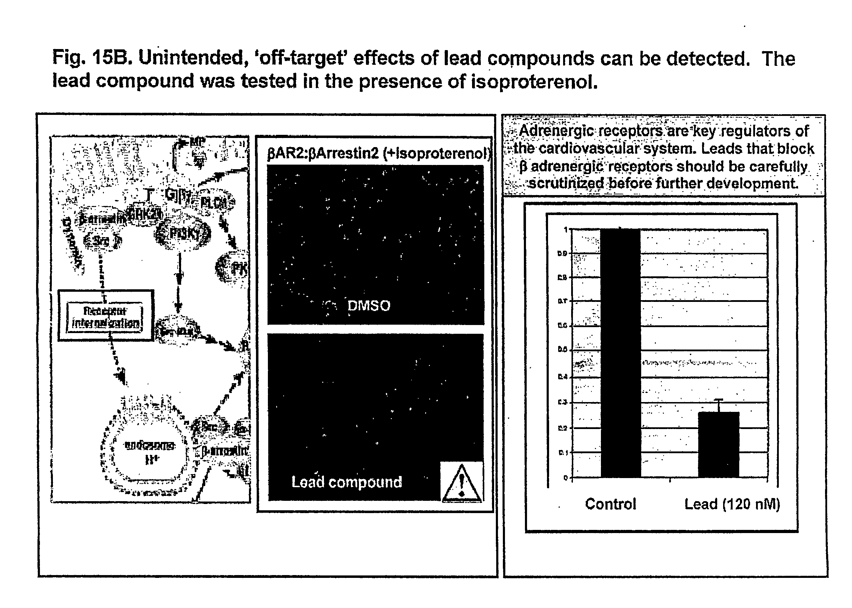

Figure 15B shows an unintended and undesirable 'off-pathway' effect of a lead compound on a GPCR pathway. The compound was a proprietary, selective and potent kinase inhibitor with antiproliferative activity, which was intended for the treatment of cancer, and was not intended or expected to have activity on GPCR pathways. The effect of the agent was observed in the presence of isoproterenol, exemplifying the utility of the pathway modulation strategy. Since the agent does not directly bind the GPCR, this unintended effect could be a result of inhibition of a GRK (G-protein receptor kinase) or other regulatory protein in this pathway. Since GPCRs regulate cardiovascular function, this 'off-pathway' activity is undesirable and this assay result allowed attrition of this compound. Other lead compounds within the same chemical series did not show this activity and thus could be pursued.

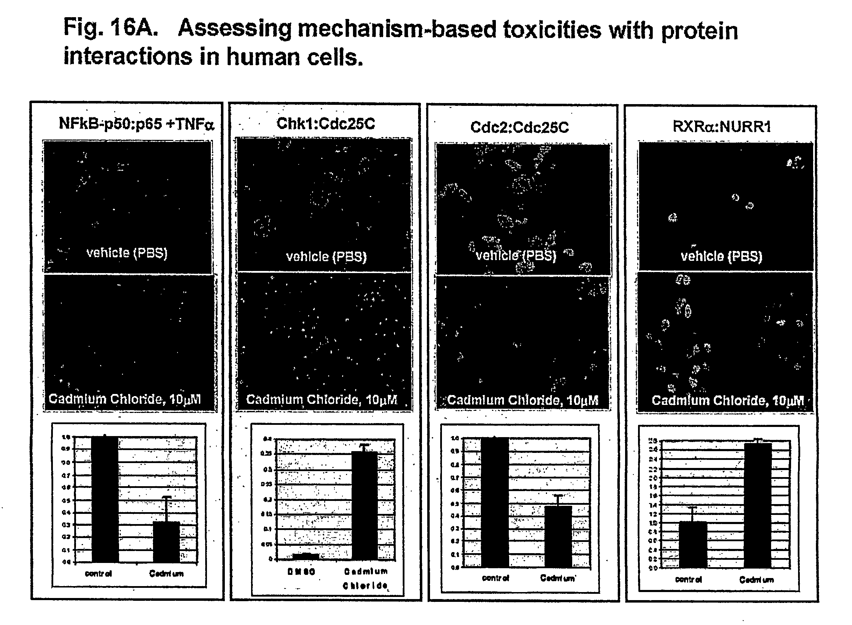

Figure 16A shows that mechanism-based toxicity can be detected in cell-based assays. The pronounced effects of a known toxicant, cadmium chloride (10 micromolar) on four different protein interactions in human cells are shown in the images and histograms. Images were taken 8 hours after cell treatment with cadmium chloride.

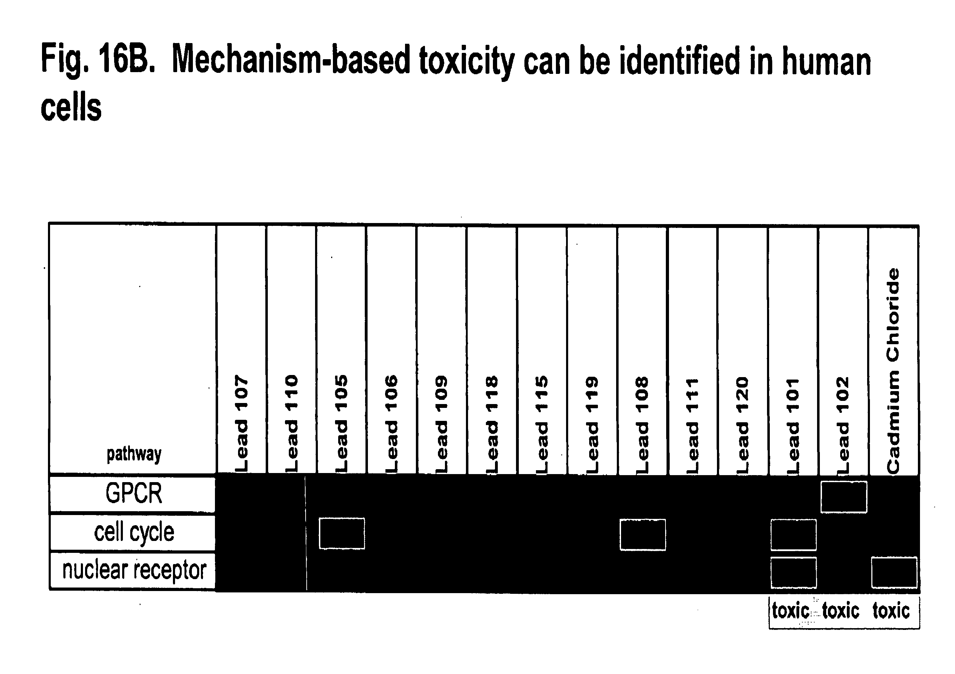

Figure 16B With the use of of assays for protein interactions, the activity profile of any novel agent can be compared with the profile of a known toxicant - such as cadmium chloride-in order to determine if the novel agent has cellular activity similar to that of the toxicant. In the example shown, four different assays were run in parallel. Green indicates an increase in the assay parameter and red indicates a decrease. In the profiles shown, two proprietary compounds - lead 101 and 102 - had profiles similar to that of cadmium and were later documented to produce hepatic toxicity in rodents. Other lead compounds in these series did not share this toxic profile.

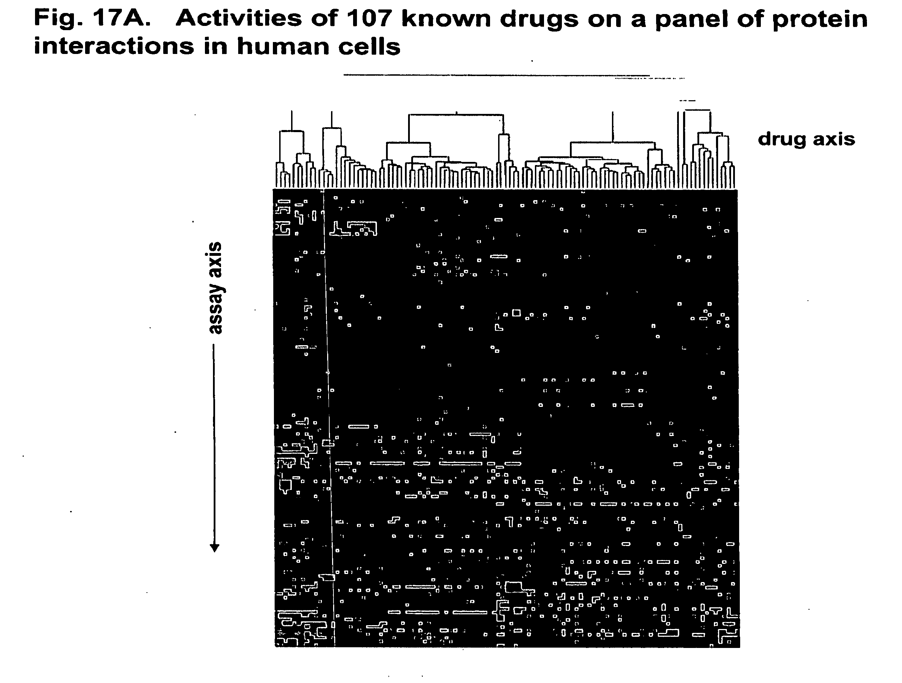

Figure 17A The activities of 107 different known drugs were assessed against a panel of 53 different protein interactions, with each interaction being tested at one or more time points in HEK293 cells with PCA. The result of each assay was coded as no effect (black), increased assay signal (green) or decreased assay signal (red). In the resulting matrix, drugs are on the x-axis and assays are on the y-axis. Each drug gave a characteristic drug signature (profile). Numerical assay results from each assay/time/pretreatment combination were clustered as described in the experimental protocol, in order to assess similarities and differences between drugs based on their activity profiles.

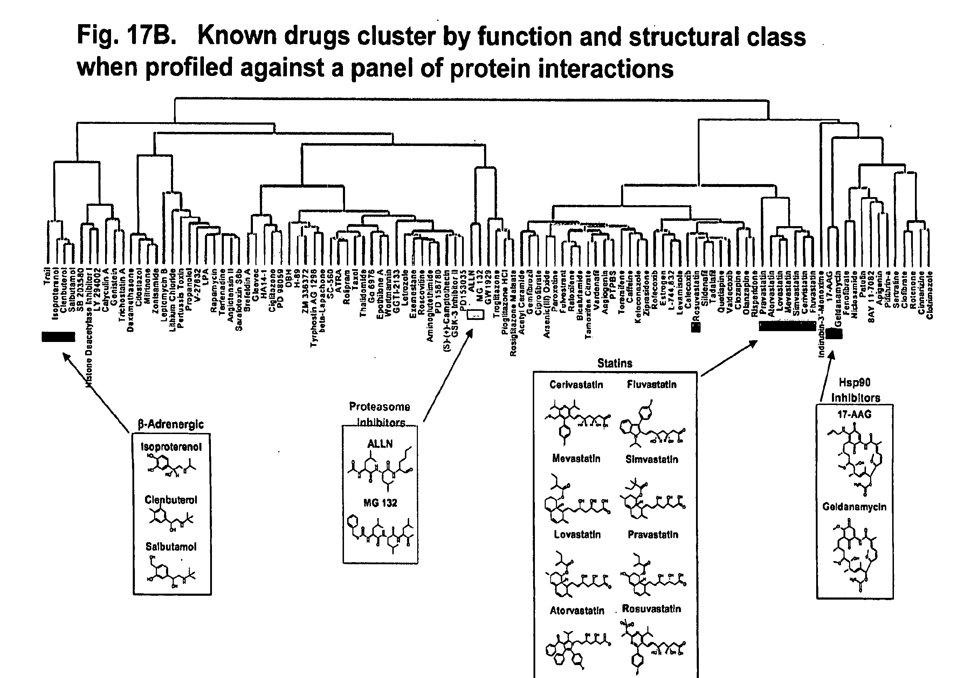

Figure 17B. The drug clusters from the x-axis of Fig. 17A are shown here with drug names attached to the dendrogram. Known drugs clustered based on their similar pathway activities, validating that the strategy faithfully captures on-pathway activities on a large scale. Differences in selectivity (off-pathway activity) between closely-related compounds were also observed, highlighting the ability of the strategy to capture off-pathway activities of a broad range of chemical compounds and targets.

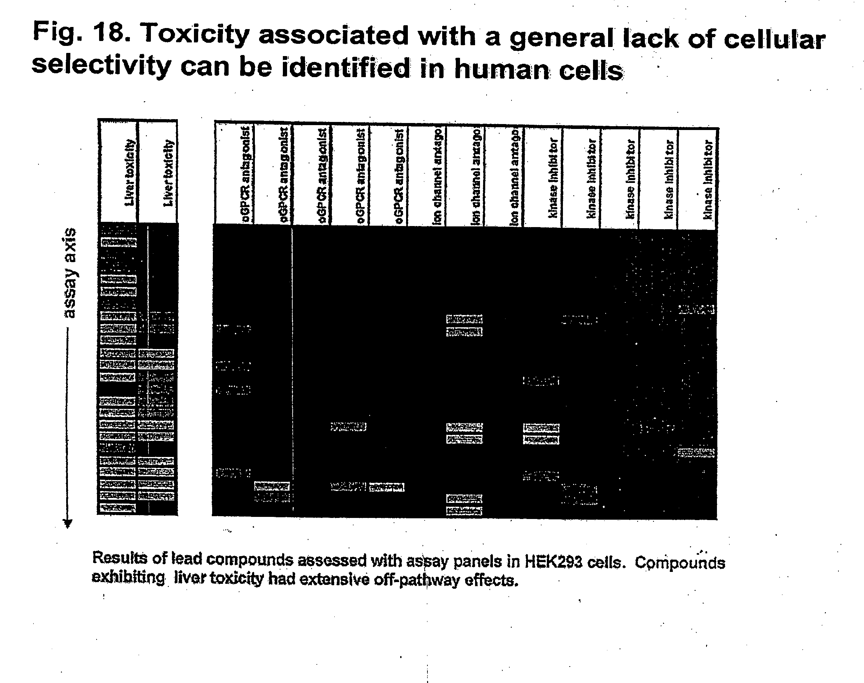

Figure 18 is a matrix showing the profiles of drug leads, representing several different drug target classes, in a panel of assays of different protein interactions. Color coding was as in Fig. 16A. Drugs that were non-selective could be distinguished readily from those that were more selective in the assay panel. Two compounds that proved to be hepatotoxic showed extensive, off-pathway activity suggestive of a general lack of selectivity in human cells. Therefore, assays of protein interactions can be used in an iterative fashion during lead optimization in order to develop leads that are 'cleaner' (more selective) in human cells. In the example shown, each drug lead was tested at a concentration that was 3x its cellular IC50; dose-response curves can also be used to compare potencies across any assay panel.

### DETAILED DESCRIPTION OF THE INVENTION

### Identifying on-pathway and off-pathway effects of drugs (pharmacological profiling)

All drugs exert their effects by acting on proteins within living cells. The typical process of drug discovery involves selecting a protein target and establishing an in vitro assay for that target of interest. The target selection phase of discovery is followed by identification, screening or development of a chemical compound that exerts the desired effect on that target. This is accomplished either by high-throughput screening of a chemical or other compound library; by crystallization of the target and in silico or wet methods to design a compound that fits into a binding site on a target; or by a combination of these and similar methods. Regardless of the method used, there is always a known target, activity, or effect of the compound. However, in nearly every case, drugs and drug candidates exert unknown effects when they contact living cells. Such unknown effects are a result of lack of specificity of the molecule in the context of the thousands of proteins making up the biochemical milieu of the living cell. In some cases, these unknown effects can result in adverse biological consequences or toxicity that is not seen until the drug is administered to hundreds or thousands of patients.

The central challenge of the pharmaceutical industry is to develop drugs that are both safe and effective in man. The balance between safety and efficacy is usually difficult to achieve as evidenced by the 75% failure rate of drugs in clinical trials. Often, a drug is completely safe but has insufficient efficacy in a particular condition to provide any appreciable benefit to the patient population for which it is intended. In other cases, a drug is efficacious but has long-term adverse effects that are not obvious until large number of patients are studied over long periods of time. Acute and chronic toxicities can lead to withdrawal of a drug from the marketplace, and there are numerous such examples that include troglitazone (Rezulin) and other drugs. Therefore, the pharmaceutical industry spends substantial research and development dollars in an attempt to understand the selectivity and potential adverse effects of drugs, prior to clinical trials. However, current methods for profiling drug selectivity are extremely limited. Therefore we sought a method to allow for the rapid identification of on-pathway and off-pathway effects of drugs on a genome-wide scale.

### Examples of intracellular proteins that form complexes

It is thought that every protein in the cell makes contacts with numerous other proteins. In addition it is believed that there are many pathways in the cell, representing organized collections of proteins that work in concert to respond to conditions or to exert a particular cellular phenotype. Figure 2 provides examples of the classes of proteins that participate in protein-protein interactions. Some of these intracellular protein-protein interactions are constitutive, that is, they do not respond to external stimuli, environmental conditions, or other modulators. By 'respond' we mean that a particular protein-protein interaction increases, decreases, or undergoes a change in subcellular distribution or pattern in response to a perturbation. However, many - if not most -interactions are modulated under certain conditions in living cells. For example, certain protein-protein interactions are induced by binding of an agonist, hormone or growth factor to a receptor which induces a signaling cascade or by a small molecule that activates an intracellular protein or enzyme. Other interactions can be inhibited, for example by binding of an antagonist or an antibody to a receptor thereby blocking a signaling cascade; by an siRNA, which silences a gene coding for a protein that is critical for a pathway; or by a drug that inhibits a particular protein within a pathway. These examples are meant to illustrate the breadth of the invention and are not limiting for the practice of the invention.

### Process for pharmacological profiling

An overview of the process of drug analysis is shown in Figure 4.

Step 1 involves selecting the chemical compounds, drug candidates or drugs to be profiled.

Step 2 involves selecting the protein-protein interactions to be tested. These may be known or novel interacting proteins. The interacting proteins can be identified, or selected, either rationally ― for example, by prior knowledge of a pathway or an interacting protein pair - or empirically. For example, the interacting proteins can be identified by one or methods that include bait-versus-library screening or pairwise (gene by gene) interaction mapping. Moreover, an unlimited number of protein-protein interaction assays can simply be constructed at random and tested empirically for their responsiveness to any number of drugs or chemical compounds and the results combined into a pharmacological profile. There is virtually no limit on the types, numbers, or identities of the protein-protein interactions that can be used in pharmacological profiling. There are likely to be hundreds of thousands of protein-protein interactions that occur in mammalian cells. However, some will be constitutive; and others will be redundant. A constitutive interaction ― which does not respond to drugs, agonists, or other perturbants ― will not be useful in pharmacological profiling. Fortunately, one can determine empirically whether a specific protein-protein interaction is useful in profiling, by simply constructing an assay for the interaction ― using one of the many assay types discussed herein - and testing it for responsiveness against a range of drugs or other compounds. A redundant interaction is one that provides information that is no different than the information provided by a different interaction. As the interactions among proteins are gradually mapped it will eventually be possible to construct a panel of completely predictive assays based on the methods provided herein. Such a panel will enable testing of any compound to determine its complete spectrum of activities against every pathway in the living cell and to determine any off-pathway activities suggestive of adverse consequences.

Step 3 of pharmacological profiling involves constructing the assays for two or more protein protein interactions. Methods suitable for constructing such assays are widespread and have been described in detail in the background of the invention; such methods, which include two-hybrid and FRET methods as well as immune capture methods, are well documented in the literature and can simply be adapted to the present invention if the interacting protein pairs are appropriately selected and the method is sensitive enough to detect and quantify changes in signal intensity or location due to the chemical compounds or drugs of interest.

In the case of fragment complementation assays, the assays are constructed according to the following scheme. For the signaling network to be studied, each of the members of a selected protein-protein pair is cloned as a gene fusion in frame (either 3' or 5') with reporter fragments that encode a PCA reporter molecule into an appropriate vector such that upon transfection into an appropriate cell line the encoded protein that is subsequently expressed in the cell carries a protein (polypeptide) fragment either at the amino or carboxy terminus of the protein encoded in the original cDNA selected. Assays can be constructed using transient transfections or stable cell lines or infection, such as with retrovirus or adenovirus designed to expressing the proteins of interest and to allow detection of the interactions of interest.

In step 4 of pharmacological profiling, each chemical compound or drug is tested against each protein-protein interaction at specific times and drug concentrations. Each drug result is compared to control (no treatment) values. (Positive and negative controls can be run for each assay, at each time point and stimulus condition). In step 5, the results of the assays are combined to establish a pharmacological profile for each compound. The results can be displayed in a variety of ways. In the examples provided herein we have used a matrix display and/or a histogram to depict the pharmacological profiles. In the case of a matrix, the results of each screen may be depicted in a color-coded matrix in which red denotes a decrease in signal intensity or location whereas green denotes an increase. Different shades of red and green can be used to depict the intensity of the change. In this manner the display is similar to that of a microarray experiment.

Software suitable for pathway analysis can be used to create pathway maps, in which the results of the pharmacological are translated into a diagram showing upstream and downstream events and linking drugs to those events. Suitable pathway analysis software is marketed by Ingenuity Systems, GeneGo, and many other providers.

### EXPERIMENTAL PROTOCOL

To apply this invention on a large scale, we constructed numerous assays for pathways controlling key cellular processes, including apoptosis, the cell cycle, DNA damage response, GPCR signalling, molecular chaperone interactions, cytoskeletal regulation, proteasomal degradation, mitogenesis, inflammation, and nuclear hormone receptor pathways. Protein-protein complexes were selected to represent interactions at different hierarchical levels within well-characterized pathways. Examples of pathways probed, and examples of assays for various targets, are provided in Figures 7-15 of this invention together with examples of expected ('on-pathway') and unexpected effects of drugs, toxicants, and lead compounds. We covered a broad range of pathways, proteins, drugs, and target classes in order to highlight the universality of the approach.

Details of particular proteins that were probed are in Tables 1-3 below. It is important to note that the invention can be used with any protein interaction so long as a dynamic assay can be constructed for that interaction. Also, particular elements of the invention, including our pathway modulation strategy for cell-based assays, can be applied to any measurement parameter of a protein complex or even an individual protein in a cell.

To assess drug effects on human signal transduction networks, we analyzed drug effects with high-content assays in a human cell line (HEK293 cells). The assays enable probing the activity of specific signaling nodes under different conditions, and activity can be monitored at temporal and spatial levels within a network of pathways. To apply this strategy on a large scale, we constructed numerous assays for pathways controlling key cellular processes, including apoptosis, the cell cycle, DNA damage response, GPCR signalling, molecular chaperone interactions, cytoskeletal regulation, proteasomal degradation, mitogenesis, inflammation, and nuclear hormone receptor pathways. By analyzing the response of diverse signaling nodes representing multiple target classes and pathways, we could pinpoint on-pathway and off-pathway drug activity and selectivity, mechanism-based and general toxicity, and drug relationships.

PCA screens employ complementary fragments (F[1] and F[2]) of a reporter protein that will fold into an active form only if fused to two proteins that interact. Protein-protein complexes (Table 1) were selected to represent known interactions that occur at different hierarchical levels within well-characterized pathways. For the examples, we engineered assays based on fragments of an intensely fluorescent mutant of YFP (Remy and Michnick, 2001; Remy and Michnick, 2004; Remy et al., 2004), which matures rapidly (9 minutes (Nagai et al., 2002), allowing for the visualization, quantification and localization of complexes formed between full-length proteins expressed at low levels in human cells. The goal of minimizing expression was to minimize potential perturbations of the endogenous pathways by the exogenous proteins (Yu, 2003).

### Fragment synthesis and construct preparation

Reporter fragments for PCA were generated by oligonucleotide synthesis (Blue Heron Biotechnology, Bothell, WA). First, oligonucleotides coding for polypeptide fragments YFP[1]and YFP[2] (corresponding to amino acids 1-158 and 159-239 of YFP) were synthesized. Next, PCR mutagenesis was used to generate the mutant fragments IFP[1] and IFP[2]. The IFP[1] fragment corresponds to YFP[1]-(F46L, F64L, M153T) and the IFP[2] fragment corresponds to YFP[2]-(V163A, S175G). These mutations have been shown to increase the fluorescence intensity of the intact YFP protein (Nagai et al., 2002). The YFP[1], YFP[2], IFP[1] and IFP[2] fragments were amplified by PCR to incorporate restriction sites and a linker sequence, described below, in configurations that would allow fusion of a gene of interest to either the 5'- or 3'-end of each reporter fragment. The reporter-linker fragment cassettes were subcloned into a mammalian expression vector (pcDNA3.1Z, Invitrogen) that had been modified to incorporate the replication origin (oriP) of the Epstein Barr virus (EBV). The oriP allows episomal replication of these modified vectors in cell lines expressing the EBNA1 gene, such as HEK293E cells (293-EBNA, Invitrogen). Additionally, these vectors still retain the SV40 origin, allowing for episomal expression in cell lines expressing the SV40 large T antigen (e.g. HEK293T, Jurkat or COS). The integrity of the mutated reporter fragments and the new replication origin were confirmed by sequencing.

PCA fusion constructs were prepared for a large number of proteins known to participate in a diverse range of cellular pathways (Table 1). The full coding sequence for each gene of interest was amplified by PCR from a sequence-verified full-length cDNA. Resulting PCR products were column purified (Centricon), digested with appropriate restriction enzymes to allow directional cloning, and fused in-frame to either the 5' or 3'-end of YFP[1], YFP[2], IFP[1] or IFP[2] through a linker encoding a flexible 10 amino acid peptide (Gly.Gly.Gly.Gly.Ser)2. The flexible linker ensures that the orientation/arrangement of the fusions is optimal to bring the reporter fragments into close proximity (Pelletier et al., 1998). Recombinants in the host strains DH5-alpha (Invitrogen, Carlsbad, CA) or XL1 Blue MR (Stratagene, La Jolla, CA) were screened by colony PCR, and clones containing inserts of the correct size were subjected to end sequencing to confirm the presence of the gene of interest and in-frame fusion to the appropriate reporter fragment. A subset of fusion constructs were selected for full-insert sequencing by primer walking. DNAs were isolated using Qiagen MaxiPrep kits (Qiagen, Chatsworth, CA). PCR was used to assess the integrity of each fusion construct, by combining the appropriate gene-specific primer with a reporter-specific primer to confirm that the correct gene-fusion was present and of the correct size with no internal deletions.

All fragments of fluorescent proteins mentioned above are the subject of commonly owned U.S. application serial number 10/724,178 filed December 1, 2003; the entire contents of which are incorporated by reference herein.

### Transient transfections

HEK293 cells were maintained in MEM alpha medium (Invitrogen) supplemented with 10% FBS (Gemini Bio-Products), 1% penicillin, and 1% streptomycin, and grown in a 37°C incubator equilibrated to 5% CO2. Approximately 24 hours prior to transfections cells were seeded into 96 well ploy-D-Lysine coated plates (Greiner) using a Multidrop 384 peristaltic pump system (Thermo Electron Corp., Waltham, Mass) at a density of 7,500 cells per well. Up to 100ng of the complementary YFP or IFP-fragment fusion vectors were co-transfected using Fugene 6 (Roche) according to the manufacturer's protocol. The list of the selected PCA pairs screened in this study, and corresponding gene and reporter fragment information, are listed in Table 2. Following 24 or 48 hours of expression, cells were screened against a panel of drugs as described below.

**Table 1. Proteins, genes, and assays for protein interactions for the present invention**

| **Assay Name** | **Gene_1** | **Gene_1 Accession** | **Gene_2** | **Gene_2 Accession** |
|---|---|---|---|---|
| α tubulin/HDAC6 | TUBA2 | NM_006001 | HDAC6 | NM_006044 |
| α-actin/α-actinin | ACTA1 | NM_001100 | ACTN1 | NM_001102 |
| AKL3L/GUK1 | AKL3L | NM_016282 | GUK1 | NM_000858 |
| AKL3L/IPP2 | AKL3L | NM_016282 | PPP1R2 | NM_006241 |
| Akt1/Cdc37 | AKT1 | NM_005163 | CDC37 | NM_007065 |
| Akt1/GSK3β | AKT1 | NM_005163 | GSK3B | NM_002093 |
| Akt1/p27 | AKT1 | NM_005163 | CDKN1B | NM_004064 |
| Akt1/p70S6K | AKT1 | NM_005163 | RPS6KB1 | NM_003161 |
| Akt1/Smad3 | AKT1 | NM_005163 | MADH3 | NM_005902 |
| AMPK/HMGCR | PRKAA1 | NM_206907 | HMGCR | NM_000859 |
| annexin I/FPRL1 | ANXA1 | NM_000700 | FPRL1 | NM_001462 |
| ARF1/gamma-COP | ARF1 | NM_001658 | COPG1 | NM_016128 |
| β2AR/β-arrestin2 | beta2AR | NM_000024 | ARRB2 | NM_004313 |
| β2AR/Gαl3 | beta2AR | NM_000024 | GNAI3 | NM_006496 |
| Bad/14-3-3σ | BAD | NM_004322 | SFN | NM_006142 |
| Bad/Bcl-xL | BAD | NM_004322 | BCL2L1 | NM_138578 |
| Bag1/Hsp70L1 | BAG1 | NM_004323 | HSP70L1 | NM_005527 |
| Bax/Bid | BAX | NM_138761 | BID | NM_001196 |
| Bcl-xL/Bik | BCL2L1 | NM_138578 | BIK | NM_001197 |
| β-arrestin2/Erk2 | ARRB2 | NM_004313 | MAPK1 | NM_002745 |
| β-arrestin2/Ub | ARRB2 | NM_004313 | UBB | NM_021009 (nt 69..296) |
| β-catenin/CBP | CTNNB1 | NM_001904 | CREBBP | S66385 (nt 1..2313) |
| β-catenin/Pin1 | CTNNB1 | NM_001904 | PIN1 | NM_006221 |
| β-TrCP/β-catenin | BTRC | NM_033637 | CTNNB1 | NM_001904 |
| β-TrCP/ROC1 | BTRC | NM_033637 | RBX1 | NM_014248 |
| c-Jun/CBP | JUN | NM_002228 | CREBBP | S66385 (nt 1..2313) |
| c-Jun/Fos | JUN | NM_002228 | FOS | NM_005252 |
| c-Src/Grk2 | CSK | NM_004383 | ADRBK1 | NM_001619 |
| Calcineurin A/Bad | PPP3CA | NM_005605 | BAD | NM_004322 |
| CaM/Calcineurin A | CALM2 | NM_001743 | PPP3CA | NM_005605 |
| CaM/DAPK2 | CALM2 | NM_001743 | DAPK2 | NM_014326 |
| CaM/MEF2C | CALM2 | NM_001743 | MEF2C | NM_002397 |
| Caspase 3/Cdc6 | CASP3 | NM_004346 | CDC6 | NM_001254 |
| Caspase 3/MST4 | CASP3 | NM_004346 | MST4 | NM_016542 |
| Caspase3/ICAD | CASP3 | NM_004346 | DFFA | NM_004401 |
| CBP/ATF-1 | CREBBP | S66385 (nt 1..2313) | ATF1 | NM_005171 |
| CBP/CBP | CREBBP | S66385 (nt 1..2313) | CREBBP | S66385 (nt 1..2313) |
| CBP/CREB1 | CREBBP | S66385 (nt 1..2313) (nt 1..2313) | CREB1 | NM_134442 |
| CCR5/PKCα | CCR5 | NM_000579 | PRKCA | NM_002737 |
| Cdc2/CyclinB | CDC2 | NM_001786 | CCNB1 | NM_031966 |
| Cdc25A/Cdc2 | CDC25A | NM_001789 | CDC2 | NM_001786 |
| Cdc25C/14-3-3ζ | CDC25C | NM_001790 | YWHAZ | NM_003406 |
| Cdc25C/Cdc2 | CDC25C | NM_001790 | CDC2 | NM_001786 |
| Cdc42/Jnk2 | CDC42 | NM_001791 | MAPK9 | NM_002752 |
| Cdc42/Pak4 | CDC42 | NM_001791 | PAK4 | NM_005884 |
| Cdc42/WASP | CDC42 | NM_001791 | WASP | NM_000377 |
| Cdk2/14-3-3σ | CDK2 | NM_001798 | SFN | NM_006142 |
| Cdk2/Axin | CDK2 | NM_001798 | Axin1 | NM_009733 |
| Cdk2/Cdc6 | CDK2 | NM_001798 | CDC6 | NM_001254 |
| Cdk2/CyclinE | CDK2 | NM_001798 | CCNE1 | NM_001238 |
| Cdk2/Mcm4 | CDK2 | NM_001798 | MCM4 | NM_005914 |
| Cdk4/Cyclin D1 | CDK4 | NM_000075 | CCND1 | NM_053056 |
| Cdk4/Rb | CDK4 | NM_000075 | RB1 | NM_000321 |
| Chk1/Cdc25A | CHEK1 | NM_001274 | CDC25A | NM_001789 |
| Chk1/Cdc25C | CHEK1 | NM_001274 | CDC25C | NM_001790 |
| Chk1/Ku70 | CHEK1 | NM_001274 | G22P1 | NM_001469 |
| Chk1/p53 | CHEK1 | NM_001274 | TP53 | NM_000546 |
| Chk2/Chk2 | CHEK2 | NM_007194 | CHEK2 | NM_007194 |
| Chk2/p53 | CHEK2 | NM_007194 | TP53 | NM_000546 |
| Crk/RalA | CRK | NM_005206 | RALA | NM_005402 |
| DGKα/c-Src | DGKA | NM_001345 | CSK | NM_004383 |
| Dnmt1/Histone H3A | Dnmt1 | NM_010066 | H3F3A | NM_002107 |
| E2F1/CEBPε | E2F1 | NM_005225 | CEBPE | NM_001805 |
| E6/E6AP | E6 | NC_001526 | E6AP | □□□□□□□□□□□□ |
| E6/p53 | E6 | NC_001526 | TP53 | NM_000546 |
| EDG2/Gγ4 | EDG2 | NM_001401 | GNG4 | NM_004485 |
| EGFR/CaM | EGFR | NM_005228 | CALM2 | NM_001743 |
| EGFR/Grb2 | EGFR | NM_005228 | GRB2 | NM_002086 |
| eIF4E/4E-BP2 | EIF4E | NM_001968 | EIF4BP2 | NM_004096 |
| eIF4E/eIF4A | EIF4E | NM_001968 | EIF4A1 | NM_001416 |
| eIF4E/eIF4G | EIF4E | NM_001968 | EIF4G1 | NM_198244 |
| EIk1/SRF | ELK1 | NM_005229 | SRF | NM_003131 |
| ERα/RXRα | ESR1 | NM_000125 | RXRA | NM_002957 |
| ERα/SRC-1 | ESR1 | NM_000125 | SRC-1 | U40396 (nt 624..1256) |
| Erk2/EIk1 | MAPK1 | NM_002745 | ELK1 | NM_005229 |
| Erk2/Mnk1 | MAPK1 | NM_002745 | MKNK1 | NM_003684 |
| Erk2/p27 | MAPK1 | NM_002745 | CDKN1B | NM_004064 |
| FAK1/Socs-3 | FAK1 | NM_005607 | Socs3 | NM_007707 |
| FXR/RXRα | Nr1h4 | NM_009108 | RXRA | NM_002957 |
| FXR/SRC-1 | Nr1h4 | NM_009108 | SRC-1 | U40396 (nt 624..1256) |
| Fz-4/Gα(o) | FZD4 | NM_012193 | Gnao1 | NM_010308 |
| Fz-4/Grk2 | FZD4 | NM_012193 | ADRBK1 | NM_001619 |
| Fz-4/RGS2 | FZD4 | NM_012193 | RGS2 | NM_002923 |
| Gαl/Gβ1 | GNAI1 | NM_002069 | Gnb1 | NM_008142 |
| GLUT4/Sumo-1 | GLUT4 | NM_001042 | SUMO1 | NM_003352 |
| Grk2/Gα(o) | ADRBK1 | NM_001619 | Gnao1 | NM_010308 |
| Grk2/Gγ2 | ADRBK1 | NM_001619 | GNG2 | XM_495987 |
| Grk2/PKCα | ADRBK1 | NM_001619 | PRKCA | NM_002737 |
| Grk2/PKCζ | ADRBK1 | NM_001619 | PRKCZ | NM_002744 |
| GRM3/Gγ4 | GRM3 | NM_000840 | GNG4 | NM_004485 |
| GSK3β/PP2A | GSK3B | NM_002093 | PPP2CA | NM_004156 |
| HDAC1/14-3-3σ | HDAC1 | NM_004964 | SFN | NM_006142 |
| HDAC1/HDAC1 | HDAC1 | NM_004964 | HDAC1 | NM_004964 |
| HDAC1/Stat1 | HDAC1 | NM_004964 | STAT1 | NM_007315 |
| Histone H3/HDAC1 | H3F3A | NM_002107 | HDAC1 | NM_004964 |
| Histone H3/Histone H3 | H3F3A | NM_002107 | H3F3A | NM_002107 |
| H-Ras/Raf1 | HRAS | NM_005343 | RAF1 | NM_002880 |
| Hsc70/p53 | HSC70 | NM_006597 | TP53 | NM_000546 |
| Hsp90β/Akt1 | HSPCB | NM_007355 | AKT1 | NM_005163 |
| Hsp90β/Bld | HSPCB | NM_007355 | BID | NM_001196 |
| Hsp90β/Cdc37 | HSPCB | NM_007355 | CDC37 | NM_007065 |
| Hsp90β/Chk1 | HSPCB | NM_007355 | CHEK1 | NM_001274 |
| Hsp90β/Eef2k | HSPCB | NM_007355 | Eef2k | NM_007908 |
| Hsp90β/Mek1 | HSPCB | NM_007355 | Map2k1 | Z16415 |
| Hsp90β/Mek2 | HSPCB | NM_007355 | Map2k2 | D14592 |
| Hsp90β/Wee1 | HSPCB | NM_007355 | Wee1 | NM_009516 |
| ICAD/CAD | DFFA | NM_004401 | DFFB | NM_004402 |
| IkBα/p65 | NFKB1A | NM_020529 | Rela | NM_009045 |
| IKKβ/IKKγ | IKBKB | NM_001556 | IKBKG | NM_003639 |
| IKKγ/p27 | IKBKG | NM_003639 | CDKNIB | NM_004064 |
| IPP2/RIPK2 | PPP1R2 | NM_006241 | RIPK2 | NM_003821 |
| ITGα5/[TGβ1 | ITGA5 | NM_002211 | ITGB1 | NM_002205 |
| Jnk1/c-Jun | MAPK8 | NM_002750 | JUN | NM_002228 |
| JNK2/ATF-2 | MAPK9 | NM_002752 | ATF2 | NM_001880 |
| Jnk2/Cdc25C | MAPK9 | NM_002752 | CDC25C | NM_001790 |
| Jnk2/Cdk2 | MAPK9 | NM_002752 | CDK2 | NM_001798 |
| Ku70/Ku80 | G22P1 | NM_001469 | XRCC5 | NM_021141 |
| Limk2/Cofilin1 | LIMK2 | NM_005569 | CFL1 | NM_005507 |
| LXRβ/RXRα | NR1H2 | NM_007121 | RXRA | NM_002957 |
| Map3k8/Clk1 | Map3k8 | NM_005204 | CLK1 | NM_004071 |
| Map3k8/PP1-r1α | Map3k8 | NM_005204 | PPP1R1A | NM_006741 |
| MAPKAPK-2/Eef2k | MAPKAPK2 | NM_032960 | Eef2k | NM_007908 |
| Mdm2/NPM1 | MDM2 | NM_002392 | NPM1 | NM_002520 |
| Mdm2/p53 | MDM2 | NM_002392 | TP53 | NM_000546 |
| Mek1/Erk2 | Map2k1 | Z16415 | MAPK1 | NM_002745 |
| Mek2/Jnk | Map2k2 | D14592 | MAPK8 | NM_002750 |
| Mevalonate | MVK | NM_000431 | MVK | NM_000431 |
| MKK6/p38α | MAP2K6 | NM_002758 | MAPK14 | NM_001315 |
| Mnk1/elF4E | MKNK1 | NM_003684 | EIF4E | NM_001968 |
| Myc/Max | MYC | NM_002467 | MAX | NM_002382 |
| NLK/c-Myb | NLK | NM_016231 | Myb | NM_010848 |
| eNos/CaM | Nos3 | NM_008713 | CALM2 | NM_001743 |
| NURR1/RXRα | NR4A2 | NM_006186 | RXRA | NM_002957 |
| Orc1/Orc2 | ORC1L | NM_004153 | ORC2L | NM_006190 |
| p21/Cdc2 | CDKN1A | NM_000389 | CDC2 | NM_001786 |
| p21/CyclinB | CDKN1A | NM_000389 | CCNB1 | NM_031966 |
| p22(phox)/p47(phox) | CYBA | NM_000101 | NOXO2 | NM_000265 |
| p27/CRM1 | CDKN1B | NM_004064 | XPO1 | NM_003400 |
| P2Y2r/Gα15 | P2RY2 | NM_002564 | GNA15 | NM_002068 |
| P2Y2r/Gγ4 | P2RY2 | NM_002564 | GNG4 | NM_004485 |
| p38α/Cdc25B | MAPK14 | NM_001315 | CDC25B | NM_021873 |
| p38α/Cdk2 | MAPK14 | NM_001315 | CDK2 | NM_001798 |
| p38α/Elk1 | MAPK14 | NM_001315 | ELK1 | NM_005229 |
| p38α/MAPKAPK-2 | MAPK14 | NM_001315 | MAPKAPK2 | NM_032960 |
| p38α/Max | MAPK14 | NM_001315 | MAX | NM_002382 |
| p38α/Mnk1 | MAPK14 | NM_001315 | MKNK1 | NM_003684 |
| p38α/Rad9 | MAPK14 | NM_001315 | RAD9 | NM_004584 |
| p53/p53 | TP53 | NM_000546 | TP53 | NM_000546 |
| p65/CBP | Rela | NM_009045 | CREBBP | S66385 (nt 1..23 1 3) |
| p65/p50 | Rela | NM_009045 | NFKB1 | NM_003998 |
| p70S6K/Map3k8 | RPS6KB1 | NM_003161 | Map3k8 | NM_005204 |
| p70S6K/RIPK2 | RPS6KB1 | NM_003161 | RIPK2 | NM_003821 |
| Pak4/Bad | PAK4 | NM_005884 | BAD | NM_004322 |
| Pak4/Cofilin | PAK4 | NM_005884 | CFL1 | NM_005507 |
| Parvin-beta/ILK | PARVB | □□□□□□□□□□□□ | ILK | NM_004517 |
| Pcna/Polδ (p50) | PCNA | NM_002592 | POLD2 | NM_006230 |
| PCNA/Xrcc1 | PCNA | NM_002592 | Xrcc1 | NM_009532 |
| Pdk1/Akt | PDPK1 | NM_002613 | AKT1 | NM_005163 |
| PFK-2/IPP2 | PFKFB1 | NM_002625 | PPP1R2 | NM_006241 |
| PIAS1/Mdm2 | PIAS1 | NM_016166 | MDM2 | NM_002392 |
| PIASy/p53 | PIASy | NM_015897 | TP53 | NM_000546 |
| PIASy/Smad3 | PIASy | NM_015897 | MADH3 | NM_005902 |
| Pin1/Cdc25C | PIN1 | NM_006221 | CDC25C | NM_001790 |
| Pin1/c-Jun | PIN1 | NM_006221 | JUN | NM_002228 |
| Pin1/p53 | PIN1 | NM_006221 | TP53 | NM_000546 |
| Pin1/p70S6K | PIN1 | NM_006221 | RPS6KB1 | NM_003161 |
| PKCα/p47Nox | PRKCA | NM_002737 | NOXO1 | NM_144603 |
| PP1cβ/HDAC1 | PPP1CB | NM_002709 | HDAC1 | NM_004964 |
| PP1-r1α/PP-1B | PPP1R1A | NM_006741 | PPP1CB | NM_002709 |
| PP2A/Cdk2 | PPP2CA | NM_U02715 | CDK2 | NM_001798 |
| PP2A/Mek1 | PPP2CA | NM_002715 | Map2k1 | Z16415 |
| PPARγ/RXRα | PPARG | NM_138712 | RXRA | NM_002957 |
| PPARγ/SRC-1 | PPARG | NM_138712 | SRC-1 | U40396 (nt 624 .1256) |
| Pthr1/Gα15 | Pthr1 | NM_011199 | GNA15 | NM_002068 |
| PTPα/c-Src | PTPRA | NM_080840 | CSK | NM_004383 |
| PTPα/Grb2 | PTPRA | NM_080840 | GRB2 | NM_002086 |
| PXR/RXRα | PXR | NM_022002 | RXRA | NM_002957 |
| PXR/SRC-1 | PXR | NM_022002 | SRC-1 | U40396 (nt624..1256) |
| Rac1/Pak1 | RAC1 | NM_006908 | PAK1 | NM_002576 |
| Rac1/Pak6 | RAC1 | NM_006908 | PAK6 | NM_020168 |
| Rad1/Rad17 | RAD1 | NM_002853 | RAD17 | NM_133338 |
| Rad9/HDAC1 | RAD9 | NM_004584 | HDAC1 | NM_004964 |
| Rad9/Hus1 | RAD9 | NM_004584 | HUS1 | NM_004507 |
| Rad9/p53 | RAD9 | NM_004584 | TP53 | NM_000546 |
| Raf1/14-3-3ε | RAF1 | NM_002880 | SFN | NM_006142 |
| Raf1/Cdc37 | RAF1 | NM_002880 | CDC37 | NM_007065 |
| Raf1/Mek1 | RAF1 | NM_002880 | Map2k1 | Z16415 |
| Raf1/Mek2 | RAF1 | NM_002880 | Map2k2 | D14592 |
| RalB/RalBP1 | RALB | NM_002881 | RALBP1 | NM_006788 |
| RARβ/RXRα | RARB | NM_000965 | RXRA | NM_002957 |
| Rb/CyclophilinA | RB1 | NM_000321 | PPIA | NM_021130 |
| RGS2/Gαi2 | RGS2 | NM_002923 | GNAI2 | NM_002070 |
| SCAP/Insig-1 | SCAP | NM_012235 | INSIG1 | NM_005542 |
| Smad3/HDAC1 | MADH3 | NM_005902 | HDAC1 | NM_004964 |
| Smurf1/Smad1 | SMURF1 | NM_181349 | MADH1 | NM_005900 |
| Speedy1/p27 | SPDY1 | NM_182756 | CDKN1B | NM_004064 |
| SRF/Sumo-1 | SRF | NM_003131 | SUMO1 | NM_003352 |
| SSTR2/Gβ1 | SSTR2 | NM_001050 | Gnb1 | NM_008142 |
| Stat1/Stat1 | STAT1 | NM_007315 | STAT1 | NM_007315 |
| Stat5a/Stat5b | STAT5A | NM_003152 | STAT5B | NM_012448 |
| STK15/Guk1 | STK15 | NM_003600 | GUK1 | NM_000858 |
| Syntaxin:Synaptobrevin2 | STX4A | NM_004604 | VAMP2 | NM_014232 |
| TFCOUP2/SRC-1 | Nr2f2 | NM_009697 | SRC-1 | U40396 (nt 624..1256) |
| TF-COUP3/TFIIB | Nr2f6 | NM_010150 | GTF2B | NM_001514 |
| THRα/RXRα | THRA | NM_003250 | RXRA | NM_002957 |
| Tlk1/Asf1 | TLK1 | NM_012290 | ASF1 | NM_014034 |
| TNFR1/FADD | TNFR1 | NM_001065 | FADD | NM_003824 |
| TNFR1/TNFR1 | TNFR1 | NM_001065 | TNFR1 | NM_001065 |
| TRAF2/p65 | TRAF2 | NM_021138 | Rela | NM_009045 |
| TRAF2/IkBa | TRAF2 | NM_021138 | NFKB1A | NM_020529 |
| \/av/Cdc42 | VAV | NM_005428 | CDC42 | NM_001791 |
| VIPR2/GβI | VIPR2 | NM_003382 | Gnb1 | NM_008142 |
| WASP/Grb2 | WASP | NM_000377 | GRB2 | NM_002086 |
| Wee1/Cdc2 | WEE1 | NM_009516 | CDC2 | NM_001786 |
| Xrcc1/Ape1 | Xrcc1 | NM_009532 | APE1 | NM_001641 |

**Table 2. Description of some of the proteins used in assay construction**

| **Gene/protein** | **Allas** | **Gene/protein** | **Allas** | **Gene/protein** | **Allas** |
|---|---|---|---|---|---|
| | | | | | |
| ACTA1 | actin, alpha 1 | Ephb4 | Htk, MDK2, Myk1, Tyro11 | PAK4 | p21(CDKN1A)-activated kinase 4 |
| ACTN1 | actinin, alpha 1 | Ephb6 | | PCTK1, isoform a | PCTAIRE1, PCTGAIRE |
| ACVR1B | | EPOR | erythropoletin receptor | PCTK1, isoform b | PCTAIRE1, PCTGAIRE |
| Acvrt1 | activin A receptor, type II-like 1 | ESR1 | ER, ESR, Era, ESRA. NR3A1 (estrogen receptor 1 (alpha) | PCTK2 | PCTAIRE protein kinase 2 |
| ADPRT | PARP | FADD | | PDGFRB | Platelet-derived growth factor receptor, beta polypeptide |
| AKT1 | PKB (protein kinase B) | FAF1 | hFAF1, CGI-03, HFAF1s: FAS-associated factor 1 | Pdk1 | death-associated protein kinase 2 |
| AKT2 | PKB (protein kinase B) | FAK1 | FAK, PTK2 | PDK2 | pyruvate dehydrogenase kinase, isoenzyme 2 |
| APC | adenomatous polyposis coli | FASTK | Fas-activated serine/threonine kinase | PDK4 | pyruvate dehydrogenase kinase, isoenzyme 4 |
| ARAF1 | PKS2, A-RAF, RAFA1 | FGFR4 | fibroblast Growth factor receptor 4 | PDPK1 | |
| ARF | | FGR | SRC2, c-fgr, p55c-fgr | PED | |
| ARHA | RhoA | FKBP | FK506 binding protein | PHKG2 | phosphorylase Kinase, gamma 2 |
| ARRB2 | ARB2, ARR2; arrestin, beta 2 | FKHRL1 | | Pik3ca | mPI3K pt10 |
| ARRB2 | beta-arrestin-2, beta arr2 | FKSG81 | | PIK3R1 | PI3K p85 |
| AT1 | AT1AR, AGTR1 | FOS | | PIM1 | PIM1 oncogene |
| Axin | | FRB | FKBP-binding domain of murine FRAP/TOR | PIM2 | proto-oncogene Pim-2 (serine threonine kinase) |
| AXL | AXL receptor tyrosine kinase | FRK | GTK, RAK, fyn-relaled kinase | Pim3 | |
| BAD | | FRS2 | SNT, SNT1, FRS2A, SNT-1, FRS2alpha, suc1-associated neurotrophic factor target (FGFR signalling adaptor) | Pkmyt1 | membrane-associated tyrosine-and threonine-specific cdc2-inhibitory kinase |
| BAK | BAK1, CDN1, BCL2L7; BCL2-antagonisit/killer 1; cell death Inhibitor 1 | G22P1 | Ku70 | PLCG | PLCG1; PLC1, PLC-II, PLC148; PLG-gamma-1 |
| BAX | | GAB1 | GRB2-associated binding protein 1 | PLK | PLK1, STPK13, polo-like-kinase |
| BC033012 | | GADD45A | | PPARG | peroxisome proliferator activated receptor gamma |
| BCL2 | B-cell lymphoma protein 2 | GADD45B | growth arrest and DNA-damage-inducible, beta | PPP2CA | PP2A, catalytic subunit |
| BCLG | apoptosis regulator BCL-G | GADD45G | growth arrest and DNA-damage-inducible, gamma | PPP2R1B | |
| BCL-X | | GNAi3 | | PRKACA | protein kinase, cAMP-dependent, catalytic, alpha |
| beta2AR | beta2-adrenergic receptor, adrb2 | GNAS1 | guanine nucleotide binding protein (G protein), alpha stimulating activity polypeptide 1 | PRKCA | |
| BID | BH3 interacting domain death agonist | Gnb1 | guanine nucleotide binding protein, beta 1 | PRKCD | Protein kinase C, delta |
| BIK | BP4, NBK, BBC1, BIP1; BCL2-Interacting killer | GNG5 | guanine nucleotide binding protein (G protein), gamma 5 | PRKCE | |
| BIM1 | | GPR34 | | Prkch | protein kinase C. eta |
| BIRC2 | API1, MIHB, clAP1, HIAP2. RNF48 | GPRK5 | | PRKCI | protein kinase C. Iota |
| BLK | B lymphoid tyrosine kinase | GPRK6 | | Prkx | protein kinase, X-linked |
| BMX | BMX non-receptor tyrosine kinase | Gprk7 | G-protein coupled receptor 7 | PTEN | |
| BRCA1 | breast cancer 1, early onset | GPRK7 | G-protein coupled receptor 7 | PTPN11 | protein tyrosine phosphatase, non-receptor type 11 |
| BTRC | b-TrCP | GRB2 | | PYK2 | PTK2, FAK, FADK, FAK1, pp125FAK |
| BTRC | b-TrCP | GRB7 | | RAC1 | rho family, small GTP binding protein Rac1 |
| BUB1B | BUBR1, Bub1A, MAD3L; BUB1 budding uninhibited by benzimidazoles 1 homolog beta (Yeast) | GS3955 | TRB2; tribbles homolog 2 | RAD1 | HRAD1; cell cycle checkpoint protein Hrad1 |
| CAMK1 | calcium/calmodulin-dependent protein kinase I | GSK3B | | Rad17 | cell cycle checkpoint protein (RAD17) |
| CAMK2G | calcium/calmodulin-dependent protein kinase 2G | H2AX | H2AFX; H2AX histone | Rad51 | RAO51 homolog (RecA homolog, E. coll) (S. cerevislae) |
| CAMK4 | calcium/calmodulin-dependent protein kinase 4 | HCK | JTK9; hemopoletic cell kinase | RAD9 | RAD9A; RAD9 homolog (S. pombe) cell cycle checkpoint control protein |
| Camkk1 | calcium/calmodulin-dependent protein kinase kinase 1 | HDAC1 | histone deacetylase 1 | RAF1 | |
| CAMKKB1 | | HIF1A | hypoxia-inducible factor 1, alpha | RALA | |
| CASP9 | | HRAS | | ralbp1 | ralA binding protein 1 |
| d-CATENIN | delta-catenin | HRI | heme-regulated initiation factor 2-alpha kinase | RAP1 | RAPGA1,KREV-1, SMGP21, RAP1GAP, KIAA0474, RAP1, GTPase activating protein 1 |
| CBP | Cyclic AMP response element binding protein | HSPA1L | hsp70-like | RARa | retinoic acid receptor alpha |
| c-CBL | | HSPB1 | HSP27, HSP28, Hsp25, HS.76067; heat shock 27kD protein 1 | RATP130CAS | |
| CCNB1 | cyclin B1 | HSPCB | hsp90, beta | RB1 | retinoblastoma |
| CCND1 | cyclinD1 | Hus1 | HUS1 checkpoint homolog (S pombe) | RBL2 | Rb2, p130 |
| CCND2 | cyclin D2 | IKBKAP | IKAP | Rela | D65. p65 NFkB |

**Table 3: Description of individual assays, and pathway modulation ('stimulation') where used**

| | **PCA Description** | **Stimulation** | **Genbank Gene 01** | **Reporter fusion orientation** | **Genbank Gene 02** | **Reporter fusion orientation** |
|---|---|---|---|---|---|---|
| 1 | 14-3-zeta:CDC25C +CPT | 500nM CPT; 16 hrs | BC003623 | C | NM 001790 | C |
| 2 | Akt1:p27 | | NM_005163 | N | NM_004064 | C |
| 3 | Akt1:PDPK1 | | NM_005163 | N | NM_002613 | C |
| 4 | BAD:BID | | NM_004322 | N | NM_001196 | C |
| 5 | bARR2: b2AR | | NM_004313 | N | NM_000024 | C |
| 6 | bARR2: b2AR+ISO | 2 M Isoproterenol; 30 min | NM_004313 | N | NM_000024 | C |
| 7 | Bcl-xL: Bad | | NM_138578 | C | NM_004322 | N |
| 8 | Bcl-xL: BIK | | NM_138578 | N | NM_001197 | N |
| 9 | Cdc2:Cdc25A +CPT | 500nM CPT; 16 hrs | NM_001786 | N | NM_001789 | C |
| 10 | Cdc2:CDC25C | | NM_001786 | N | NM_001790 | C |
| 11 | Cdc2:CDC25C +CPT | 500nM CPT; 16 hrs | NM_001786 | N | NM_001790 | c |
| 12 | Cdc2:Wee1 | | NM_001786 | N | NM_009516 | N |
| 13 | CDC42:PAK4 | | NM_001791 | N | NM_005884 | C |
| 14 | Chk1:CDC25A +CPT | 500nM CPT; 16 hrs | NM_001274 | N | NM_001789 | C |
| 15 | Chk1:CDC25C | | NM_001274 | N | NM_001790 | C |
| 16 | Chk1:CDC25C +CPT | 500nM CPT; 16 hrs | NM_001274 | N | NM_001790 | C |
| 17 | Cofilin:LIMK2 | | NM_005507 | C | NM_005569 | N |
| 18 | CvclinB:Cdc2 | | NM_031966 | N | NM_001786 | C |
| 19 | CyclinD:Cdk4 | | NM_053056 | N | NM_001791 | C |
| 20 | CyclinE:Cdk2 | | NM_001238 | N | NM_001798 | N |
| 21 | E6:E6AP | | AJ388069 | .N | NM_130839 (CDS 1729..2028) | C |
| 22 | E6:p53 | | AJ388069 | N | NM_000546 | N |
| 23 | Elk1:MAPK1 | | NM_005229 | C | NM_002745 | C |
| 24 | ESR1:SRC-1 | | NM_000125 | N | U40396 (CDS 624..1256) | N |
| 25 | Gai:b2AR | | NM_006496 | C | NM 000024 | C |
| 26 | H-Ras:Raf | | NM_005343 | N | NM 002880 | C |
| 27 | Hsp90:CDC37 | | NM_007355 | C | NM 007065 | N |
| 28 | Hsp90:Eef2k | | NM_007355 | C | NM 007908 | N |
| 29 | Hsp90:MEK1 | | NM_007355 | C | Z16415 | N |
| 30 | MAPK9:ATF2 | | L31951 | N | NM 001880 | N |
| 31 | Mdm2:p53 | | BC009893 | N | NM 000546 | C |
| 32 | MKNK1:MAPK1 | | NM_003684 | C | NM 002745 | C |
| 33 | MAX: MYC | | NM_002382 | C | NM 002467 | C |
| 34 | ntCBP:JUN | | S66385 | N | NM 002228 | C |
| | | | (CDS 1..2313) | | | |
| 35 | ntCBP:p65 | | S66385 (CDS 1..2313) | N | NM_009045 | N |
| 36 | Cdc2: p21 | | NM_001786 | N | NM 000389 | N |
| 37 | P27:UbiquitinC | | NM_004064 | N | NM_021009 (CDS 69..296) | N |
| 38 | p50:p65 | | NM_003998 | N | NM_009045 | N |
| 39 | p53:Chk1 | | NM_000546 | C | NM 001274 | N |
| 40 | p53:Chk1 +CPT | 500nM CPT; 16 hrs | NM_000546 | C | NM 001274 | N |
| 41 | p53:p53 | | NM_000546 | C | NM 000546 | C |
| 42 | p53:p53 +CPT | 500nM CPT; 16 hrs | NM_000546 | C | NM_000546 | C |
| 43 | PAK4:Cofilin | | NM_005884 | C | NM_005507 | C |
| 44 | Pin1:CDC25C | | NM_006221 | C | NM_001790 | C |
| 45 | Pin1:JUN | | NM_006221 | C | NM_002228 | C |
| 46 | Pin1:p53 | | NM_006221 | C | NM_000546 | C |
| 47 | PXR:RXRa | | BC017304 | C | NM_002957 | C |
| 48 | RAD9:p38a | | NM_004584 | C | NM_001315 | N |
| 49 | RAD9:p53 | | NM_004584 | c | NM 000546 | N |
| 50 | Raf1:Map2k2 | | NM_002880 | C | D14592 | N |
| 51 | RB1:CDK4 | | BC040540 | C | NM_001791 | C |
| 52 | RXRa:PPARg | | NM-002957 | C | NM_138711 | C |
| 53 | Smad3:HDAC | | NM_005902 | N | NM 004964 | C |

**Table 4: Drugs and concentrations used in pharmacological profiling example**

| **DRUG** | **Source** | **Concentration** | **DRUG** | **Source** | **Concentration** | **DRUG** | **Source** | **Concentration** |
|---|---|---|---|---|---|---|---|---|
| (S)-(+)-Camptothecin | Sigma | 500 nM | GGTI-2133 | Calbiochem | 5 microM | Quetiapinc | Sequoia | 2 microM |
| 17-AAG | Tocris | 5 microM | Gleevec | Novartis | 10 microM | Raloxifene | LKT Labs, Inc. | 500 nM |
| Acetyl ceramide | Sigma | 10 microM | Gö 6976 | Calbiochem | 100.00 nM | Rapamycin | Calbiochem | 250 nM |
| ALLN | Calbiochem | 25 microM | GSK-3 Inh. II | Calbiochem | 1 microM | Risperidone | Sequoia | 2 microM |
| Aminoglutethimide | Microsource | 30 microM | GW1929 | Alexis | 3 microM | Rofecoxib | Sequoia | 10 microM |
| Angiogenin | Sigma | 100 ng/ml | H-89 | Calbiochem | 2 microM | Rolipram | Calbiochem | 25 microM |
| Angiotensin II | Calbiochem | 300 nM | HA14-1 | Tocris | 2 microM | Roscovitine | Calbiochem | 5 microM |
| Apigenin | Calbiochem | 50 microM | HDAC Inh. I | Calbiochem | 10 microM | Rosiglitazone | LKT Labs, Inc. | 15 microM |
| Arsenic(III) Oxide | Sigma | 5 microM | Indirubin-3'-Monoxime | Calbiochem | 10 microM | Rosuvastatin | Sequoia | 30 microM |
| ATRA | Sigma | 5 microM | Isoproterenol | Sigma | 2 microM | Rotenone | Sigma | 300 nM |
| BAY 11-7082 | Calbiochem | 10 microM | Ketoconazole | Sigma | 30 microM | Salbutamol | Sigma | 2 microM |
| bicalutamide | Sequoia | 500 nM | L-744,832 | Sigma | 10 microM | Sarafotoxin S6b | Calbiochem | 100 nM |
| Brefeldin A | Sigma | 50 mg/ml | LeptomycinB | Sigma | 10.00 ng/ml | SB 203580 | Calbiochem | 25 microM |
| Caffeine | Sigma | 50 microM | Letrozole | Sequoia | 1.50 microm | SC-560 | Calbiochem | 250 nM |
| Calyculin A | Calbiochem | 2nM | Lithium Chloride | Sigma | 1000 microM | Sildenafil | Sequoia | 1 microM |
| Celecoxib | Sequoia | 10 microM | Lovastatin | Calbiochem | 30 microM | Simvastatin | Calbiochem | 30 microM |
| cerivistatin | Sequoia | 30 microM | LPA | Sigma | 5 microM | Tadalafil | Sequoia | 1 microM |
| Ciglitazone | Calbiochem | 15 microM | LY 294002 | Calbiochem | 25 microM | Tamoxifen | Calbiochem | 500 nM |
| Cilostazol | Sigma | 2 microM | Mevastatin | Calbiochem | 30 microM | Taxol | Calbiochem | 2.5 microM |
| Ciprofibrate | Sigma | 30 microM | MG 132 | Tocris | 1 microM | Thalidomide | Calbiochem | 250 microM |
| Clenbuterol | Sigma | 2 microM | Milrinone | Sigma | 200 nM | Toremifene | Sequoia | 500 nM |
| Clofibrate | Sigma | 30 microM | Olanzapine | Sequoia | 2 microM | TRAIL | Sigma | 50.00 ng/ml |
| Clozapine | Sequoia | 2 microM | Paroxetine | Sequoia | 10 microM | Trichostatin A | Calbiochem | 5 microM |
| DBH | Calbiochem | 5 microM | Patulin | Sigma | 10 microM | Troglitazone | Calbiochem | 15 microM |
| Dexamethasone | Sigma | 500 nM | PD 158780 | Calbiochem | 1 microM | Tyrphostin AG 1296 | Calbiochem | 5 microM |
| Epothilone A | Calbiochem | 100 nM | PD 98059 | Calbiochem | 20 microM | Tyrphostin AG 1433 | Calbiochem | 25 microM |
| Estrogen | Calbiochem | 500 nM | PD153035 | Calbiochem | 200 nM | Valdecoxib | Sigma | 10 microM |
| Exemestane | Sequoia | 1.50 microM | Pertussis Toxin | Sigma | 100 ng/ml | Vardenafil | Sequoia | 1 microM |
| Fluvastatin | Calbiochem | 30 microM | Pifithrin-a | Calbiochem | 50 microM | Wortmannin | Calbiochem | 500 nM |
| fulvestrant | Tocris | 500 nM | Pioglitazone | Calbiochem | 15 microM | Y-27632 | Calbiochem | 25 microM |
| Geldanamycin | Calbiochem | 2.5 microM | Pravastatin | Calbiochem | 30 microM | Ziprasidone | Sequoia | 2 microM |
| Gemfibrozil | Sigma | 30 microM | Propanolol | Calbiochem | 2 microM | ZM 336372 | Calbiochem | 5 microM |
| Genistein | Calbiochem | 12.5 microM | PTPBS | Calbiochem | 500 nM | | | |

### Stable cell lines

For several interactions, stable cell lines were generated. For p50/p65 and IkBa/p65, HEK293 cells were transfected with the YFP[2]-p65 fusion vector and stable cell lines were selected using 100 µg/ml Hygromycin B (Invitrogen). Selected cell lines were subsequently transfected with YFP[1]-p50 or IκBα-YFP[1], and stable cell lines expressing YFP[1]-p50/YFP[2]-p65 and IκBα-YFP[1]/ YFP[2]-p65 were isolated following double antibiotic selection with 50 µg/ml Hygromycin B and 500 µg/ml Zeocin. For b2AR/B-arrestin, HEK293 cells were transfected with the YFP[1]-β-arrestin fusion vector and stable cell lines were selected using 1000 µg/ml Zeocin. Selected cell lines were subsequently transfected with the beta2-AR-YFP[2] fusion vector and stable cell lines expressing YFP[1]-beta-arrestin/beta2-AR-YFP[2] were isolated following double antibiotic selection with 200 µg/ml Hygromycin B and 500 µg/ml Zeocin. For Akt1/PDK1, HEK293 cells were co-transfected with the YFP[1]-Akt1 and PDK1-YFP[2] fusion vectors and stable cell lines were selected using 1000 µg/ml Zeocin. For all cell lines, the fluorescence signals were stable over at least 25 passages (data not shown). Approximately 24 hours prior to drug treatments, cells were seeded into 96 well ploy-D-Lysine coated plates (Greiner) using a Multidrop 384 peristaltic pump system (Thermo Electron Corp., Waltham, Mass).

### Drug study

HEK293 cells (7,500 per well) were co-transfected with up to 100ng of the complementary fusion vectors using Fugene 6 (Roche) according to the manufacturer's protocol. 24 or 48 hours after transfection, cells were screened against drugs in duplicate wells as described below. For drug studies with p50:p65, betaArr2:beta2AR and Akt1:Pdk1, stable cell lines were seeded 24 hrs prior to drug treatment.

107 different drugs (names and doses are listed in Table 4) were tested against 49 distinct assays in duplicate at one or more time points. Drug concentrations were chosen to approximate three times (3X) the published cellular IC₅₀s and were tested to ensure lack of toxicity in HEK293 cells. All liquid handling steps were performed using a Biomek FX (Beckman Instruments, Fullerton, CA). Cells expressing the PCA pairs were incubated in cell culture medium containing drugs for 30 min., 90 min., and 8 hours, or - in the case of DNA damage response pathways - for 18 hours. For certain pathways, cells were first treated with drugs and then stimulated with agonist (CPT, TNFalpha or isoproterenol) to induce the pathway. In this manner, drugs that block an agonist-dependent pathway could be identified. Following drug treatments cells were simultaneously stained with 33 µg/ml Hoechst 33342 (Molecular Probes) and 15 µg/ml TexasRed-conjugated Wheat Germ Agglutinin (WGA; Molecular Probes), and fixed with 2% formaldehyde (Ted Pella) for 10 minutes. Cells were subsequently rinsed with HBSS (Invitrogen) and maintained in the same buffer during image acquisition.

### Fluorescence image analysis

Cell images were acquired with a Discovery-1 automated fluorescence imager (Molecular Devices, Inc.) equipped with a robotic arm (CRS Catalyst Express; Thermo Electron Corp., Waltham, Mass). The following filter sets were used to obtain images of 4 non-overlapping populations of cells per well: excitation filter 480+/-40nm, emission filter 535+/-50nm (YFP); excitation filter 360+/-40nm, emission filter 465+/-30nm (Hoechst); excitation filter 560+/- 50nm, emission filter 650+/-40nm (Texas Red; drug study). Four scans were acquired for each well with each scan representing 150-300 cells. A constant exposure time for each wavelength was used to acquire all images for a given assay.

Raw images in 16-bit grayscale TIFF format were analyzed using modules from the ImageJ API/library (http://rsb.info.nih.gov/ij/, NIH, MD). First, images from each fluorescence channel (Hoechst, YFP and Texas Red; the latter only for the drug study) were normalized using the ImageJ built-in rolling-ball algorithm. (Sternberg, 1983). Since each PCA generates signal in a specific subcellular compartment or organelle, and treatment with a drug may effect a change in complex localization or signal intensity, different algorithms were required to accurately quantitate fluorescent signals localized to the membrane, nucleus or cytosol. Each assay was categorized according to the subcellular localization of the fluorescent signal, and changes in signal intensity across each sample population were quantified using one of four automated image analysis algorithms.

Algorithm 1: Changes in mean fluorescence throughout the entire cell were quantified after the generation of masks based on auto- thresholded Hoechst (nuclear stain), Texas red (WGA; membrane stain) or YFP (PCA signal) images. A histogram obtained from the un-thresholded YFP image (YI) was used to define global background (the mean of the lowest intensity peak).Overlay of all three masks defined individual particles (which approximate cells). Positive pixels within the YI were sampled to calculate the mean pixel intensity for all positive particles within a scan (positive particle mean, PPM).

Algorithm 2: To measure changes in fluorescence restricted to the nucleus, all pixels above a user-defined gating value that fell within the nuclear mask were sampled from the YI to determine mean nuclear fluorescence and total nuclear fluorescence (normalized to Hoechst area) resulting in NucSum or Nuc Mean.

Algorithm 3: To measure population changes in total fluorescence, all pixels above a user-defined gating value were sampled from the YI to determine mean total fluorescence and total fluorescence (normalized to Hoechst area) resulting in Bulk Sum or Bulk Mean. Algorithm 4: To measure changes in fluorescence at the membrane, a mask based on the thresholded Texas red (WGA) image was used to sample pixels within the membrane region. Membrane-associated pixels above a manually established gating value were sampled to determine mean membrane fluorescence.

For all algorithms, data were corrected for global background before calculating mean values. For each assay test sets of images were generated. Each set included images of cells treated with vehicle alone (control) and images of cells treated with a signature compound (known modulator of the assay). The fluorescence in the images were quantified with each algorithm, and the results were compared to manual scores of the images to identify the algorithm best suited to quantify differences between the treated sample and control.

The effect of each treatment on each assay was compared to the result for the vehicle control (PBS for isoproterenol and propranolol; DMSO for all other drugs). For data generated by algorithm 1, the PPM was pooled for each data point (PCA pair/stimulus/drug) from at least 8 scans. An outlier filter was applied to exclude those particles falling outside the range of the group (mean ± 3SD) prior to calculating the PPM for the entire sample. For algorithms 2 -4, the Nuc Sum , Bulk Sum or Mem Sum for each sample represents the mean from at least 8 scans for drugs after application of a 2SD filter to exclude scans with fluorescent artifacts. Results of the drug study were repeated in at least three separate experiments for 'signature' drugs and showed concordance between the repeat experiments.

### Matrices and Clustering

In the matrices, each row represents a unique drug and each column represents a unique assay at a particular time point in the absence or presence of a pathway modulator such as TNFalpha, CPT or isoproterenol. Each data point was formed by taking the log of the ratio of the sample to the control. Every row and column carries equal weight. The Ward hierarchical clustering algorithm (Ward, 1963) and Euclidean distance metrics (http://www.r-project.org/) were used for clustering the treatments. For display purposes, each data point within the matrix is color- coded to illustrate relative differences within an assay. An increase relative to the control value is displayed as green and a decrease is displayed as red. Each color is further divided into two levels: level 1 (2x CV), or level 2 (1.5x CV). Level one is displayed as the brighter hue and level 2 as the darker hue.

### Results

### Scope of the invention

We have shown that this invention provides a universal method for pharmacological profiling and drug discovery, that can be applied to any test compound, drug, toxicant, drug target, pathway or cell of interest. Numerous examples are shown in Figures 6-16 of a large range of proteins and pathways.

The invention allows the identification of on-pathway and off-pathway effects of drugs and allows for the improvement of drug candidates through lead optimization and selective attrition. The invention can be applied in an iterative fashion to assess the properties of drug leads, modify those properties, and re-test the modified leads. With this approach, cellular structure-activity studies are possible wherein the structures of chemical compounds are linked to particular assay results and pharmacological profiles, allowing the identification of desired and undesired chemical constituents. In this manner, the invention is an example of chemical genomics, as it allows for the detection of chemical effects on a genome-wide scale in a biological system. The invention may be used with cell-based or in vitro assays, although cell based assays, as used herein, require less manipulation.

### On-pathway and off-pathway effects of drugs, toxicants, and lead compounds

Figure 6(A-B) show examples of interactions in GPCR pathways. Figure 7(A-C) shows examples of interactions in the ubiquitin/sumo/proteasome pathways and drug effects on those pathways. Figure 8(A-D) shows examples of interactions in kinase signalling pathways, and drug effects on those pathways. Figure 9 (A-B) shows examples of interactions in heat shock protein pathways, and drug effects on those pathways. Figure 10 (A-B) shows examples of interactions in nuclear hormone receptor pathways, and drug effects on those pathways. Figure 11 (A-B) shows examples of interactions in apoptotic pathways, and drug effects on those pathways. Figure 12 shows additional examples of drug effects (in the absence or presence of pathway modulators) on various pathways. Figure 13(A-B) shows pharmacological profiles depicted as histograms. Figure 14 illustrates the pathway modulation strategy with results for DNA damage response pathways. Figure 15 (A-B) illustrates the pathway modulation strategy with results for a GPCR pathway. These examples were selected to represent known drug target classes; these examples, and the interactions listed in Table 1, are provided to illustrate the breadth of the invention, and are not intended to limit the scope or use of the invention. The invention can be applied to any protein in any pathway. Examples of protein interactions and drug effects are discussed below.

With this strategy, drug effects can be seen within minutes or hours of drug treatment, allowing the determination of both short term and long term effects. Fig. 12 (A-D) shows representative images for 17 known protein complexes that are modulated by known mechanisms, including post-translational modifications, protein degradation or stabilization or protein translocation. Each drug shown caused either an increase, decrease, or a shift in the subcellular location of the signal for a particular complex at a particular time of treatment compared to the appropriate vehicle control. Images of various complexes show the predominantly membrane, nuclear and cytoplasmic locations of the respective complexes, highlighting the high-content nature of these assays and the ability to resolve subcellular localization of signals with automated microscopy.

For example, many kinases interact with chaperones such as Hsp90 and Cdc37 (Perdew et al., 1997; Stancato et al., 1993, Arlander et al., 2003). Images of such interactions are shown in Fig. 12 (A). Pharmacological inhibitors of HSPs (geldanamycin or 17-allylamino-geldanamycin [17=AAG]) caused the loss of numerous complexes involving client proteins. For example, Akt1:Hsp90beta, Akt1:p27, Cdc37:Akt1 and Akt1:Pdk1 were all sensitive to geldanamycin and 17-AAG as were H-Ras:Raf-1, Chk1:Cdc25C, Cdc2:Cdc25C; Cdc2:Wee1; and Chk1:p53. Examples of the effects of geldanamycin and 17AAG on Akt1 complexes are shown in Fig. 12 (A) and in Fig. 9B. Thus the physical and functional associations between targets, as well as specific pharmacological effects on the targets, can be determined by treating live cells with drugs and monitoring the protein complexes within pathways of interest.

In another example, the beta2 adrenergic receptor (beta2AR) interacts with beta-arrestin (beta-ARR2) following ligand-induced phosphorylation of the receptor by GPCR kinases (GRKs )(Lin et al., 2002). In the absence of agonist, there were no visible betaArr2:beta2AR complexes (Figure 15 left panel). Treatment of cells with the adrenergic agonist, isoproterenol, caused the formation of numerous betaArr2:beta2AR complexes within minutes (Figure 15 right panel). The fluorescence micrograph in Fig. 15 shows a punctate cytoplasmic pattern that is consistent with the time-course of binding of arrestin to the receptor and subsequent internalization via clathrin-coated pits (Zhang et al., 1996). Pretreatment of cells with the inverse agonist, propranolol, prevented the isoproterenol-induced interaction and internalization of betaArr2:beta2AR as would be predicted (Zhang et al., 1996). These results highlight the ability of this approach to detect temporal effects of agonists and antagonists directly on their known targets. Since many GPCRs couple to beta-arrestin, these and any other downstream interactions can be probed with this strategy.

A key feature of the invention is the ability to distinguish effects of different drugs at different levels of pathway hierarchy, as illustrated in the following example. In proliferating cells, phosphatidylinositol-3- kinase (PI3K) recruits Pdk and Akt to the cell membrane (Anderson et al., 1998). Wortmannin and LY294002 are known inhibitors of PI3Ks (Vlahos et al., 1994), which prevent the synthesis of phosphoinositide (PIP₃) membrane receptors for Akt, thus preventing the translocation of Akt to the membrane where it is phosphorylated and activated by phosphatidylinositol-3-dependent kinase (Pdkl). We studied the effects of drugs on pathways involving Akt by assessing the complex between Akt1 and phosphatidylinositol-3-dependent kinase (Pdk1) and between Akt1 and the cyclin-dependent kinase inhibitor, p27 (CDKN1B; Kip1), which plays a role in cell cycle regulation. In control wells, Akt1:Pdk1 complexes were predominantly localized at the cell membrane (Fig. 8C) whereas Akt1:p27 complexes were concentrated in the cell nucleus (Fig. 9B). Wortmannin and LY294002 caused a rapid re-localization of Ak1t:Pdk1 from the membrane to the cytoplasm and a decrease in total Akt÷1:Pdk1 complexes (Fig. 8C) but had no significant effect on Aktl:p27 (not shown). However, both complexes were sensitive to geldanamycin and 17-AAG. These results suggest that the pool of Akt1:p27complexes in the nucleus is insensitive to drugs that act at an early stage of Akt signalling. The distinct locations and responses of Akt1:Pdk1 and Aktl:p27 illustrate that each assay reports on the biology of a particular Akt complex rather than the total cellular pool of Akt, a feature crucial to detecting unique effects of drugs on distinct cellular processes and compartments. Kinase inhibitors with upstream effects can be detected by probing interactions downstream. This is shown in Fig. 12D, wherein LY294002 induced p53:p53 complexes in the presence of CPT, and in Fig. 8D, wherein two different nonspecific kinase inhibitors reduced the H-Ras1:Raf-1 complexes. These results both validate the utility of the network profiling strategy that is diagrammed in Fig. 3 and show that on-pathway as well as off-pathway effects can be detected with the invention.

G-protein-coupled receptors (GPCRs) represent a major druggable target class of proteins, as do cytokine/growth factor receptors, and nuclear hormone receptors. Nuclear hormone receptors modulate the effects of steroids such as estrogen and testosterone, the effects of retinoids, and of synthetic agents such as the thiazolidinediones and fibrates. Agonists of nuclear hormone receptors are marketed for a variety of indications including the treatment of metabolic disease, diabetes, and hyperlipidemia. Figure 10A illustrates some of the interactions in these pathways and the effects of a known agonist, rosiglitazone (marketed as Avandia) on its receptor target, PPARgamma. Such assays can be used to probe any number of known or orphan receptors and to determine if test compounds activate or inhibit these interactions. In addition, known agonists such as rosiglitazone can be used in conjunction with the pathway modulation strategy diagrammed in Fig. 14A to identify agents that block these activated pathways.

In addition to the chaperone-, GPCR-, nuclear hormone- and kinase-mediated pathways discussed above, Figure 12 shows examples of complexes involved in dynamic GTPase-mediated processes, the cytoskeleton, the cell cycle, apoptosis, DNA damage response pathways and nuclear hormone receptor interactions. Representative effects of drugs on these pathways as detected by these reporter complexes are described here.

GTPase interactions with effector proteins are recognized as key molecular switches. Fig. 8D and Fig. 13A illustrates a prototypical GTPase:kinase effector complex (H-Ras:Rafl) that was sensitive to non-selective kinase inhibitors, including BAY 11-7082. BAY 11-7082, originally identified as an IKK inhibitor, also blocked the translocation of p50:p65 (NFkappaB) from the cytoplasm to the nucleus in response to TNFalpha, as would be expected (Fig. 12B) (Thevenod et al., 2000). With respect to pathways controlling cytoskeletal morphology, LIM kinase 2 (Limk2) inactivates the actin depolymerizing factor Cofilin (Sumi et al., 1999). Cofilin:Limk2 complexes were almost completely eliminated by the kinase inhibitor indirubin-3'-monoxime (Fig. 12C). Cell cycle proteins that were studied included Cdc25C, Cdk4, cyclin D1 and p53. Treatment with the proteasome inhibitor, ALLN, resulted in the gradual accumulation of Cyclin D1:Cdk4 in the nucleus (Fig. 12B and 13B), consistent with the regulation of cyclin D1 levels by the 26S proteasome (Diehl et al., 1997). Isoproterenol induced Mnk1:Erk2, a result that is consistent with previous reports of adrenergic agonist-induced MAPK activity (Fig.12D) (Daaka et al., 1997; Muhlenbeck et al., 1998). As mentioned above, LY294002 increased p53:p53, consistent with reports that Akt phosphorylates and activates Mdm2, enhancing the ubiquitination and degradation of p53 (Ogawara et al., 2002). Finally, complexes of the ligand-activated transcription factors, PPARgamma:SRC-1(Fig. 12B) and PPARgamma:RXRalpha (Fig. 10A) increased in response to treatment with the known PPARgamma agonist, rosiglitazone, as would be predicted (Nolte et al., 1998).

We also observed interesting effects of particular drugs that are novel but are consistent with published reports of pathway organization. The protein cytokine TRAIL, which is a TNFalpha-family ligand (Wiley et al., 1995) increased Bcl-xL:Bad (Fig. 12B). Also, the protein kinase inhibitor, Gö6976, increased complexes of PAK4 with the cytoskeletal protein cofilin (Fig. 12D). In yeast, Cdc42 and PAK homologues are activated, and cytoskeletal changes occur, as cells progress through the G2/M phase of the cell cycle (Richman et al., 1999). Gö6976 is a potent inhibitor of checkpoint (Chk) kinases (Kohn et al., 2003), and facilitates progression through G2/M. Thus, Gö6976-mediated activation of processes downstream from Cdc42/PAK, such as PAK4:Cofilin, is not surprising. We also demonstrate for the first time images of a nuclear complex containing a transcription factor (c-Jun) and a prolyl isomerase (Pin1). Pinl binds to Jun, after it is phosphorylated and activated by the kinase JNK -(Wulf et al., 2001) We observed strong inhibition of Pin1:Jun complexes following cell treatment with clofibrate (Fig. 12D). Clofibrate, a PPARalpha agonist, has been shown to increase the association of PPARalpha with its transcriptional co-regulators, and to reduce the transcriptional activity of c-Jun (Yokoyama et al., 1993) leading to the suggestion by these authors of a direct interaction of PPARgamma and c-Jun. In addition, PPARgamma agonists have been shown to inhibit activity of the JNK pathway (Irukayama-Tomobe et al., 2004), which would lead to decreased Pinl:Jun complexes, as we observed.

In sum, both expected and unexpected drug effects on a wide range of target classes and cellular processes could be detected at short and/or long time points by monitoring protein complexes and their responses to drug treatment.

### Chemically related drugs had common activities in the cellular assay panel

The examples presented above illustrate that individual effects of drugs on cellular pathways and processes can be determined by analyzing the spatial and temporal dynamics of protein complexes. We extended this approach to pharmacological profiling of 107 different drugs (Table 4) targeting 6 therapeutic areas (cancer, inflammation, cardiovascular disease, diabetes, neurological disorders, infectious disease) or that function as general modulators of cellular mechanisms (e.g. protein transport). We focused on physiologically relevant drug concentrations to avoid widespread off-target effects often observed with super-physiological compound dosing. Drugs were tested at multiple time points ranging from 30 minutes to 16 hours in order to capture early temporal effects on cellular pathways. As an example, some compounds elicit a steady increase in assays signals over time whereas others cause a biphasic change in signal intensity, and other drug treatments result in decreases in signal intensity over time.

Quantitative data for all drug/assay results are displayed in a color-coded matrix clustered for both drugs and individual assay time points (Fig. 17A) and an expanded view of the drug cluster dendrogram is shown in Figure 17B. Remarkably, the drugs clustered primarily to known structure-target classes, in spite of the fact that the assays were not intentionally selected to report on pathways targeted by the drugs. The results suggest that shared drug effects on cellular processes could be elucidated by assessing protein complex dynamics within the highly ramified cellular signalling networks. Groups of structurally related drugs, with reportedly similar or identical cellular targets, generated functional clusters (Fig. 17B). These included the proteasome inhibitors ALLN and MG-132; the beta-adrenergic GPCR agonists, isoproterenol, clenbuterol, and salbutamol; the HSP90 inhibitors, 17-AAG and geldanamycin; and the PPARgamma agonists, pioglitazone, rosiglitazone and troglitazone. In these and other examples, similarities in pharmacological profiles, which led to the observed clusters, were associated with shared structural features within drug groups.

Known toxicants can be used in conjunction with this invention, both to study mechanisms underlying toxicity and to establish pharmacological profiles related to specific toxic mechanisms. Lead compounds and other test agents can then be compared to the known toxicants to determine if the test compound has similar activities. For example, cadmium chloride had pronounced effects on multiple pathways, as shown in Fig. 16A. Some of these are known or expected, such as the effect of cadmium on NFkappaB (p50:p65), and both the expected and unexpected effects of cadmium could be pinpointed in these assays. The profiles of lead compounds were compared with the profiles of cadmium, as shown in Fig. 16B. Two proprietary chemical leads had profiles that were similar to that of cadmium. These compounds were later documented to produce hepatic toxicity in rodents. Pharmacological profiling of the leads according to this invention would have identified those activities prior to animal studies, saving time and money in preclinical trials.

Some activities of drugs are desired in certain contexts, but undesirable in other contexts. The invention can be used to identify these activities. Fig. 15B shows that a proprietary lead compound, originally designed as a kinase inhibitor for the oncology clinic, had a potent effect on the beta-adrenergic receptor. This activity was undesired in light of the intended indication, and allowed this compound to be attritted. Later studies showed that this compound was indeed cardiotoxic, thereby validating the utility of the invention and of the protein-protein interactions provided herein and the methods for their use.

This result also highlights an important aspect and general strategy of this invention which we call a 'pathway modulation strategy' (Fig. 14A). Many cellular pathways have low activity in a basal or unstimulated state, but can be activated with one or more agonists or activators. If a cell is first treated with a test compound and then treated with an agonist or activator, the ability of the test compound to block pathway activation can be determined. This is the case for the lead compound studied in Fig 15B, which blocked the beta-adrenergic receptor; an effect which could only be seen in the presence of isoproterenol. (In the absence of isoproterenol, this pathway is completely shut off, as shown in the upper left image of Fig. 15B). Further, agents that block the TNF-dependent pathway could be seen by treating with the agent and then stimulating cells with TNF (Fig. 12).

The pathway modulation strategy is further exemplified in Fig. 14B, wherein cells were treated with test compounds (drugs) and then with camptothecin. Camptothecin (CPT) induces DNA damage over a period of time, and induces various interactions in damage response pathways, as shown in the images of Fig. 14B. Evaluation of the effects of various compounds in the presence of CPT allowed for the detection of agents that either block the effects of CPT or potentiate the effects of CPT. Some of these effects will be either desired or undesired depending on the context. For example, inhibitors of checkpoint kinases are in development for use in the oncology clinic in conjunction with DNA damaging agents. For such agents, synergistic effects with CPT are desired and can be detected with this strategy. Taxol and terfenadine activated many of these pathways, as shown in the matrix. In contrast, geldanamycin, 17-AAG and Y27632 had opposite effects on several of these pathways.

As shown in Fig. 14B, Terfenadine activated several DNA damage response pathways in a manner similar to that found for paclitaxel (Taxol) a known and effective anti-cancer agent. This prompted an evaluation of the potential anti-proliferative activities of terfenadine. Studies of terfenadine in a prostate carcinoma cell line showed that, consistent with its pharmacological profile as determined herein, it had antiproliferative activity (data not shown). Therefore, pharmacological profiling of known drugs according to this invention may be used to rapidly identify potential new uses, indications, and combinations of known drugs for human therapeutics. This is accomplished by performing pharmacological profiling with a known drug and comparing the resulting profile to that of other agents with known therapeutic properties. If the test agent has a profile similar to that of an agent with a known/desired profile then the test agent can be assessed in functional assays to determine if it has the same functions as the comparator drug.

Whereas some cellular pathways are 'off' in the basal state but can be turned 'on' by treating cells with pathway agonists, other cellular pathways may be 'on' in the basal state but can be turned 'off' by treating cells with antagonists or inhibitors. For example, Akt is concentrated in the membrane, and Akt1:Pdk1 is 'on', in HEK293 cells grown in the presence of serum, as shown by the robust membrane signal for Akt1:Pdk1 in the basal state (Fig. 8C left panel). Thus, cells expressing Akt1:Pdk1 could be treated with antagonists or inhibitors, such as wortmannin or Ly294002, in conjunction with the test agent of interest. If the test agent is capable of reversing the effect of the antagonist or inhibitor then it would restore the Akt1:Pdk1 signal to the membrane (basal) state. The invention thus provides for treating cells with a test compound of interest in conjunction with another agent. The additional agent (pathway modulator) can be applied either before, after, or simultaneously with the application of the test compound of interest, with the optimal protocol being determined empirically for each pathway and assay. It will be appreciated by one skilled in the art that the pathway modulation strategy for pharmacological profiling is a unique component of the invention that is not limited to the measurement of a protein-protein interaction but can be applied to other measurements of pathway activity, including as the amount or subcellular location of any individual protein or of its post-translational modification status.

It will be appreciated to one skilled in the art that the protein kinase, Aktl, undergoes the same pattern of subcellular redistribution in response to wortmannin or Ly294002 as we observe for the Akt1:PDK1 complex. Therefore, assays capable of measuring the subcellular location and redistribution of Akt can be used as an alternative to assays measuring the subcellular location and redistribution of the Akt1:Pdk1 complex. Assays for the redistribution of Akt are commercially available from BioImage (Denmark) and may be used in pharmacological profiling in conjunction with this invention. Similarly, assays for the redistribution of the p65 subunit of the NFkappaB complex may be used in conjunction with this invention, as an alternative to the measurement of the p50:p65 complex, and will give substantially similar results. Such assays are available either from BioImage, where the assay is based on the expression of a GFP-tagged p65 protein, or from Cellomics Inc, where the assay is based on immunofluorescence with a p65-specific antibody. In a final example, the subcellular location of beta-arrestin shifts to the endosomes in response to beta-adrenergic agonists and antagonists; these assays are known as TransFluor assays (Norak/Molecular Devices) and may be used as an alternative to the measurement of the receptor: arrestin complex shown in Fig. 15A. This invention provides for the use of high-content assay panels for pharmacological profiling, and such panels may include high-content assays for the redistribution of individual proteins in addition to high-content or high-throughput assays for protein complexes or interactions.

Our invention also addresses several limitations of previously utilized pharmacological profiling strategies such as gene chips. A feature of the invention is the ability to capture both short-term and longer-term effects at multiple points within a pathway. Some of the observed assay dynamics were responses to secondary or functional cellular activities, such as apoptosis or cell cycle progression, particularly at the longer (8-hour or 16-hour) time points, whereas the shorter time points report on more immediate effects of the compounds. By probing signalling at multiple time points, and at multiple levels within pathway hierarchies, we can identify changes directly involving or proximal to the target of interest.

Approaches such as this are necessary to clarify the functional and biochemical roles of particular proteins, and to identify the most promising targets for development of human therapeutics. A rapid exclusion of compounds with potential deleterious effects (the so-called "fail-fast" strategy) is equally important. In addition, understanding the specific biochemical nature of the off-target activity would enable refinement of a chemical structure to address desirable or undesirable functional attributes of a molecule. The strategy described here provides an efficient means to flag compounds for exclusion from further studies, or to redirect development to other therapeutic areas.

The entire contents including the references cited therein of the following patents and publications are incorporated by reference in their entirety for all purposes to the same extent as if each individual patent, patent application or publication were so individually denoted.
US 6,372,431 Cunningham, et al.
US 6,801,859 Friend, et al.
US 6,673,554 Kauvar, et al.
US 6,270,964 Michnick, et al.
US 6,294,330 Michnick, et al.
US 6,428,951 Michnick, et al.
US Patent Application 20030108869 Michnick, et al.
US Patent Application 20020064769 Michnick, et al.
US 6,342,345 Blau, et al.
US 5,891,646 Barak, et al.
US 6,110,693 Barak, et al.
US 6,518,021 Thastrup, et al.
US 5,583,217 Quante, et al.
US 5,516,902 Quante, et al.
US 5,514,561 Quante, et al.
US 5,338, 843 Quante, et al.
Alessi, D. R., Cuenda, A., Cohen, P., Dudley, D. T., and Saltiel, A. R. (1995). PD 098059 is a specific inhibitor of the activation of mitogen-activated protein kinase kinase in vitro and in vivo. J Biol Chem 270, 27489-27494.
Anderson, K. E., Coadwell, J., Stephens, L. R., and Hawkins, P. T. (1998). Translocation of PDK-1 to the plasma membrane is important in allowing PDK-1 to activate protein kinase B. Curr Biol 8, 684-691.
Angers et al., 2000, Detection of beta-2-adrenergic receptor dimerization in living cells using bioluminescence resonance energy transfer, Proc. Natl. Acad. Sci. USA 97: 3684-3689.
Arlander, S. J., Eapen, A. K., Vroman, B. T., McDonald, R. J., Toft, D. O., and Karnitz, L. M. (2003). Hsp90 inhibition depletes Chk1 and sensitizes tumor cells to replication stress. J Biol Chem 278, 52572-52577.
Basso, A. D., Solit, D. B., Chiosis, G., Giri, B., Tsichlis, P., and Rosen, N. (2002). Akt forms an intracellular complex with heat shock protein 90 (Hsp90) and Cdc37 and is destabilized by inhibitors of Hsp90 function. J Biol Chem 277, 39858-39866.
Bouwmeester, T., Bauch, A., Ruffner, H., Angrand, P. O., Bergamini, G., Croughton, K., Cruciat, C., Eberhard, D., Gagneur, J., Ghidelli, S., et al. (2004). A physical and functional map of the human TNF-alpha/NF-kappa B signal transduction pathway. Nat Cell Biol 6, 97-105.
Brazil, D. P., Park, J., and Hemmings, B. A. (2002). PKB binding proteins. Getting in on the Akt. Cell 11 1, 293-303.
Daaka, Y., Luttrell, L. M., and Lefkowitz, R. J. (1997). Switching of the coupling of the beta2-adrenergic receptor to different G proteins by protein kinase A. Nature 390, 88-91.
Dai, K., Kobayashi, R., and Beach, D. (1996). Physical interaction of mammalian CDC37 with CDK4. J Biol Chem 271, 22030-22034.
de Ruiter, N. D., Wolthuis, R. M., van Dam, H., Burgering, B. M., and Bos, J. L. (2000). Ras-dependent regulation of c-Jun phosphorylation is mediated by the Ral guanine nucleotide exchange factor-Ral pathway. Mol Cell Biol 20, 8480-8488.
Dempsey, P. W., Doyle, S. E., He, J. Q., and Cheng, G. (2003). The signaling adaptors and pathways activated by TNF superfamily. Cytokine Growth Factor Rev 14, 193-209.
Diehl, J. A., Zindy, F., and Sherr, C. J. (1997). Inhibition of cyclin D1 phosphorylation on threonine-286 prevents its rapid degradation via the ubiquitin-proteasome pathway. Genes Dev 11, 957-972.
Fry, D. W., Kraker, A. J., McMichael, A., Ambroso, L. A., Nelson, J. M., Leopold, W. R., Connors, R. W., and Bridges, A. J. (1994). A specific inhibitor of the epidermal growth factor receptor tyrosine kinase. Science 265, 1093-1095.
Galarneau, A., Primeau, M., Trudeau, L.-E. and Michnick, S.W. (2002). A Protein fragment Complementation Assay based on TEM1 ß-lactamase for detection of protein-protein interactions. Nat Biotechnol, 20: 619-622.
Gardner, O. S., Dewar, B. J., Earp, H. S., Samet, J. M., and Graves, L. M. (2003). Dependence of peroxisome proliferator-activated receptor ligand-induced mitogen-activated protein kinase signaling on epidermal growth factor receptor transactivation. J Biol Chem 278, 46261-46269.
Giaever, G., Flaherty, P., Kumm, J., Proctor, M., Nislow, C., Jaramillo, D. F., Chu, A. M., Jordan, M. I., Arkin, A. P., and Davis, R. W. (2004). Chemogenomic profiling: identifying the functional interactions of small molecules in yeast. Proc Natl Acad Sci U S A 101, 793-798.
Giaever, G., Shoemaker, D. D., Jones, T. W., Liang, H., Winzeler, E. A., Astromoff, A., and Davis, R. W. (1999). Genomic profiling of drug sensitivities via induced haploinsufficiency. Nat Genet 21, 278-283.
Gong, L., Li, Y., Nedeljkovic-Kurepa, A., and Sarkar, F. H. (2003). Inactivation of NF-kappaB by genistein is mediated via Akt signaling pathway in breast cancer cells. Oncogene 22, 4702-4709.
Hong, S., Lee, H. Y., Lee, Y. W., and Lee, H. W. (2000). Apicidin, a histone deacetylase inhibitor, inhibits proliferation of tumor cells via induction of p21WAF1/Cip1 and gelsolin. Cancer Res 60, 6068-6074.
Hanke, J. H., Gardner, J. P., Dow, R. L., Changelian, P. S., Brissette, W. H., Weringer, E. J., Pollok, B. A., and Connelly, P. A. (1996). Discovery of a novel, potent, and Src family-selective tyrosine kinase inhibitor. Study of Lck- and FynT-dependent T cell activation. J Biol Chem 271, 695-701.
Hazra, S., Xiong, S., Wang, J., Rippe, R. A., Krishna, V., Chatterjee, K., and Tsukamoto, H. (2004). Peroxisome proliferator-activated receptor gamma induces a phenotypic switch from activated to quiescent hepatic stellate cells. J Biol Chem 279, 11392-11401.
Hu, E., Kim, J. B., Sarraf, P., and Spiegelman, B. M. (1996). Inhibition of adipogenesis through MAP kinase-mediated phosphorylation of PPARgamma. Science 274, 2100-2103.
Huang J, Z. H., Haggarty SJ, Spring DR, Hwang H, Jin F, Snyder M, Schreiber SL (2004). Finding new components of the target of rapamycin (TOR) signaling network through chemical genetics and proteome chips. ProcNatlAcadSciUSA 101, 16594-16599.
Huh, W. K., Falvo, J. V., Gerke, L. C., Carroll, A. S., Howson, R. W., Weissman, J. S., and O'Shea, E. K. (2003). Global analysis of protein localization in budding yeast. Nature 425, 686-691.
Ihmels J, F. G., Bergmann S, Sarig O, Ziv Y, Barkai N (2002). Revealing modular organization in the yeast transcriptional network. NatGenet 31, 370-377.
Irukayama-Tomobe, Y., Miyauchi, T., Sakai, S., Kasuya, Y., Ogata, T., Takanashi, M., Iemitsu, M., Sudo, T., Goto, K., and Yamaguchi, I. (2004). Endothelin-1-induced cardiac hypertrophy is inhibited by activation of peroxisome proliferator-activated receptor-alpha partly via blockade of c-Jun NH2-terminal kinase pathway. Circulation 109, 904-910.
Ito, A., Kawaguchi, Y., Lai, C. H., Kovacs, J. J., Higashimoto, Y., Appella, E., and Yao, T. P. (2002). MDM2-HDACl-mediated deacetylation of p53 is required for its degradation. Embo J 21, 6236-6245.
Kimura, Y., Rutherford, S. L., Miyata, Y., Yahara, I., Freeman, B. C., Yue, L., Morimoto, R. I., and Lindquist, S. (1997). Cdc37 is a molecular chaperone with specific functions in signal transduction. Genes Dev 11, 1775-1785.
Kohn, E. A., Yoo, C. J., and Eastman, A. (2003). The protein kinase C inhibitor Go6976 is a potent inhibitor of DNA damage-induced S and G2 cell cycle checkpoints. Cancer Res 63, 31-35.
Kunath, T., Gish, G., Lickert, H., Jones, N., Pawson, T., and Rossant, J. (2003). Transgenic RNA interference in ES cell-derived embryos recapitulates a genetic null phenotype. Nat Biotechnol 21, 559-561.
Lamphere, L., Fiore, F., Xu, X., Brizuela, L., Keezer, S., Sardet, C., Draetta, G. F., and Gyuris, J. (1997). Interaction between Cdc37 and Cdk4 in human cells. Oncogene 14, 1999-2004. Li, W., Zhu, T., and Guan, K. L. (2004). Transformation potential of Ras isoforms correlates with activation of phosphatidylinositol 3-kinase but not ERK. J Biol Chem 279, 37398-37406. Li, Y., and Sarkar, F. H. (2002). Inhibition of nuclear factor kappaB activation in PC3 cells by genistein is mediated via Akt signaling pathway. Clin Cancer Res 8, 2369-2377.
Lin, F. T., Chen, W., Shenoy, S., Cong, M., Exum, S. T., and Lefkowitz, R. J. (2002). Phosphorylation of beta-arrestin2 regulates its function in internalization of beta(2)-adrenergic receptors. Biochemistry 41, 10692-10699.
Losel, R., and Wehling, M. (2003). Nongenomic actions of steroid hormones. Nat Rev Mol Cell Biol 4, 46-56.
Lum PY, A. C., Stepaniants SB, Cavet G, Wolf MK, Butler JS, Hinshaw JC, Garnier P, Prestwich GD, Leonardson A, Garrett-Engele P, Rush CM, Bard M, Schimmack G, Phillips JW, Roberts CJ, Shoemaker DD (2004). Discovering modes of action for therapeutic compounds using a genome-wide screen of yeast heterozygotes. Cell 116, 121-137.
Lum, P. Y., Armour, C. D., Step aniants, S. B., Cavet, G., Wolf, M. K., Butler, J. S., Hinshaw, J. C., Gamier, P., Prestwich, G. D., Leonardson, A., et al. (2004). Discovering modes of action for therapeutic compounds using a genome-wide screen of yeast heterozygotes. Cell 116, 121-137.
MacLean, M., and Picard, D. (2003). Cdc37 goes beyond Hsp90 and kinases. Cell Stress Chaperones 8, 114-119.
Mei, S., Ho, A. D., and Mahlknecht, U. (2004). Role of histone deacetylase inhibitors in the treatment of cancer (Review). Int J Oncol 25, 1509-1519.
Michnick, S.W., Remy, I., C.-Valois, F.X., Vallee-Belisle, A., Galarneau, A. and Pelletier, J.N. (2000) Detection of Protein-Protein Interactions by Protein Fragment Complementation Strategies, Parts A and B (John N. Abelson, Scott D Emr and Jeremy Thorner, editors) A Volume of Methods in Enzymology. 328, 208-230.
Ming, X. F., Viswambharan, H., Barandier, C., Ruffieux, J., Kaibuchi, K., Rusconi, S., and Yang, Z. (2002). Rho GTPase/Rho kinase negatively regulates endothelial nitric oxide synthase phosphorylation through the inhibition of protein kinase B/Akt in human endothelial cells. Mol Cell Biol 22, 8467-8477.
Mitsiades, C. S., Mitsiades, N., and Koutsilieris, M. (2004). The Akt pathway: molecular targets for anti-cancer drug development. Curr Cancer Drug Targets 4, 235-256.
Muhlenbeck, F., Haas, E., Schwenzer, R., Schubert, G., Grell, M., Smith, C., Scheurich, P., and Wajant, H. (1998). TRAIL/Apo2L activates c-Jun NH2-terminal kinase (JNK) via caspase-dependent and caspase-independent pathways. J Biol Chem 273, 33091-33098.
Nagai, T., Ibata, K., Park, E. S., Kubota, M., Mikoshiba, K., and Miyawaki, A. (2002). A variant of yellow fluorescent protein with fast and efficient maturation for cell-biological applications. Nat Biotechnol 20, 87-90.
Nolte, R. T., Wisely, G. B., Westin, S., Cobb, J. E., Lambert, M. H., Kurokawa, R., Rosenfeld, M. G., Willson, T. M., Glass, C. K., and Milburn, M. V. (1998). Ligand binding and co-activator assembly of the peroxisome proliferator-activated receptor-gamma. Nature 395, 137-143.
Ochel, H. J., Schulte, T. W., Nguyen, P., Trepel, J., and Neckers, L. (1999). The benzoquinone ansamycin geldanamycin stimulates proteolytic degradation of focal adhesion kinase. Mol Genet Metab 66, 24-30.
Ogawara, Y., Kishishita, S., Obata, T., Isazawa, Y., Suzuki, T., Tanaka, K., Masuyama, N., and Gotoh, Y. (2002). Akt enhances Mdm2-mediated ubiquitination and degradation of p53. J Biol Chem 277, 21843-21850.
Park, A., and Baichwal, V. R. (1996). Systematic mutational analysis of the death domain of the tumor necrosis factor receptor 1-associated protein TRADD. J Biol Chem 271, 9858-9862.
Parsons AB, B. R., Ding H, Li Z, Zhang C, Sheikh B, Brown GW, Kane PM, Hughes TR, Boone C (2004). Integration of chemical-genetic and genetic interaction data links bioactive compounds to cellular target pathways. NatBiotechnol 22, 62-69.
Pelletier, J. N., Campbell-Valois, F. X., and Michnick, S. W. (1998). Oligomerization domain-directed reassembly of active dihydrofolate reductase from rationally designed fragments. Proc Natl Acad Sci U S A 95, 12141-12146.
Pelletier, J.N., Remy, I. and Michnick, S.W. (1998). Protein-Fragment Complementation Assays: a General Strategy for the in vivo Detection of Protein-Protein Interactions. Journal of Biomolecular Techniques, 10: 32-39.
Perdew, G. H., Wiegand, H., Vanden Heuvel, J. P., Mitchell, C., and Singh, S. S. (1997). A 50 kilodalton protein associated with raf and pp60(v-src) protein kinases is a mammalian homolog of the cell cycle control protein cdc37. Biochemistry 36, 3600-3607.
Perlman, Z. E., Slack, M. D., Feng, Y., Mitchison, T. J., Wu, L. F., and Altschuler, S. J. (2004). Multidimensional drug profiling by automated microscopy. Science 306, 1194-1198.
Phelan, M. L., and Nock, S. (2003). Generation ofbioreagents for protein chips. Proteomics 3, 2123-2134.
Picard, D. (2002). Heat-shock protein 90, a chaperone for folding and regulation. Cell Mol Life Sci 59, 1640-1648.
Pruitt, K., and Der, C. J. (2001). Ras and Rho regulation of the cell cycle and oncogenesis. Cancer Lett 171, 1-10.
Remy, I., Wilson, I.A. and Michnick, S.W. (1999). Erythropoietin receptor activation by a ligand-induced conformation change. Science, 283: 990-993.
Remy, I., and Michnick, S. W. (2001). Visualization of biochemical networks in living cells. Proc Natl Acad Sci U S A 98, 7678-7683.
Remy, I., and Michnick, S. W. (2003). Dynamic visualization of expressed gene networks. J Cell Physiol 196, 419-429.
Remy, I., and Michnick, S. W. (2004). Regulation of apoptosis by the Ftl protein, a new modulator of protein kinase B/Akt. Mol Cell Biol 24, 1493-1504.
Remy, I., Montmarquette, A., and Michnick, S. W. (2004). PKB/Akt modulates TGF-beta signalling through a direct interaction with Smad3. Nat Cell Biol 6, 358-365.
Remy, I. and Michnick, S.W. (1999). Clonal Selection and In Vivo Quantitation of Protein Interactions with Protein Fragment Complementation Assays. Proc Natl Acad Sci USA, 96: 5394-5399.
Remy, I., Pelletier, J.N., Galarneau, A. and Michnick, S.W. (2002). Protein Interactions and Library Screening with Protein Fragment Complementation Strategies. Protein-protein interactions: A molecular cloning manual. E.A. Golemis, editor. Cold Spring Harbor Laboratory Press. Chapter 25, 449-475.
Richman, T. J., Sawyer, M. M., and Johnson, D. I. (1999). The Cdc42p GTPase is involved in a G2/M morphogenetic checkpoint regulating the apical-isotropic switch and nuclear division in yeast. J Biol Chem 274, 16861-16870.
Rodriguez-Viciana, P., Sabatier, C., and McCormick, F. (2004). Signaling specificity by Ras family GTPases is determined by the full spectrum of effectors they regulate. Mol Cell Biol 24, 4943-4954.
Rossi, et al., Monitoring protein-protein interactions in intact eukaryotic cells by beta-galactosidase complementation, Proc Natl Acad Sci USA 94: 8405-8410, 1997; and US 6,342,345
Ruehr et al., 1999, Cyclic AMP-dependent protein kinase binding to A-kinase anchoring proteins in living cells by fluorescence resonance energy transfer of green fluorescent protein fusion proteins, J. Biol. Chem. 274: 33092- 33096)
Schmid, J.A., et al. (2000) Dynamics of NFkappaB and IkappaBalpha studied with green fluorescent protein (GFP) fusion proteins. J. Biol. Chem. 275 (22): 17035-17042.
Semizarov, D., Kroeger, P., and Fesik, S. (2004). siRNA-mediated gene silencing: a global genome view. Nucleic Acids Res 32,3836-3845.
Shoemaker, D. D., and Linsley, P. S. (2002). Recent developments in DNA microarrays. Curr Opin Microbiol 5, 334-337.
Stancato, L. F., Chow, Y. H., Hutchison, K. A., Perdew, G. H., Jove, R., and Pratt, W. B. (1993). Raf exists in a native heterocomplex with hsp90 and p50 that can be reconstituted in a cell-free system. J Biol Chem 268, 21711-21716.
Sternberg, S. R. (1983). Biomedical image processing. IEEE Computer 16, 22-34. Subramaniam, R., Desveaux, D., Spickler, C., Michnick, S. W., and Brisson, N. (2001). Direct visualization of protein interactions in plant cells. Nat Biotechnol 19, 769-772.
Sumi, T., Matsumoto, K., Takai, Y., and Nakamura, T. (1999). Cofilin phosphorylation and actin cytoskeletal dynamics regulated by rho- and Cdc42-activated LIM-kinase 2. J Cell Biol 147, 1519-1532.
Teruel, T., Hernandez, R., Benito, M., and Lorenzo, M. (2003). Rosiglitazone and retinoic acid induce uncoupling protein-1 (UCP-1) in a p38 mitogen-activated protein kinase-dependent manner in fetal primary brown adipocytes. J Biol Chem 278, 263-269.
Thevenod, F., Friedmann, J. M., Katsen, A. D., and Hauser, I. A. (2000). Up-regulation of multidrug resistance P-glycoprotein via nuclear factor-kappaB activation protects kidney proximal tubule cells from cadmium- and reactive oxygen species-induced apoptosis. J Biol Chem 275, 1887-1896.
Varley, C. L., Stahlschmidt, J., Lee, W. C., Holder, J., Diggle, C., Selby, P. J., Trejdosiewicz, L. K., and Southgate, J. (2004). Role of PPARgamma and EGFR signalling in the urothelial terminal differentiation programme. J Cell Sci 117, 2029-2036.
Vlahos, C. J., Matter, W. F., Hui, K. Y., and Brown, R. F. (1994). A specific inhibitor of phosphatidylinositol 3-kinase, 2-(4-morpholinyl)-8-phenyl-4H-1-benzopyran-4-one (LY294002). J Biol Chem 269, 5241-5248.
Ward, J. (1963). Hierarchical grouping to optimize an objective function. Journal of the American Statistical Association 58, 236--244.
Westwick, J. K., Cox, A. D., Der, C. J., Cobb, M. H., Hibi, M., Karin, M., and Brenner, D. A. (1994). Oncogenic Ras activates c-Jun via a separate pathway from the activation of extracellular signal-regulated kinases. Proc Natl Acad Sci U S A 91, 603 0-6034.
Westwick, J. K., Lambert, Q. T., Clark, G. J., Symons, M., Van Aelst, L., Pestell, R. G., and Der, C. J. (1997). Rac regulation of transformation, gene expression, and actin organization by multiple, PAK-independent pathways. Mol Cell Biol 17, 13 24-1335.
White, M. A., Nicolette, C., Minden, A., Polverino, A., Van Aelst, L., Karin, M., and Wigler, M. H. (1995). Multiple Ras functions can contribute to mammalian cell transformation. Cell 80, 533-541.
Wiley, S. R., Schooley, K., Smolak, P. J., Din, W. S., Huang, C. P., Nicholl, J. K., Sutherland, G. R., Smith, T. D., Rauch, C., Smith, C. A., and et al. (1995). Identification and characterization of a new member of the TNF family that induces apoptosis. Immunity 3, 673-682.
Wulf, G. M., Ryo, A., Wulf, G. G., Lee, S. W., Niu, T., Petkova, V., and Lu, K. P. (2001). Pin1 is overexpressed in breast cancer and cooperates with Ras signaling in increasing the transcriptional activity of c-Jun towards cyclin D1. Embo J 20, 3459-3472.
Yokoyama, Y., Tsuchida, S., Hatayama, I., and Sato, K. (1993). Lack of peroxisomal enzyme inducibility in rat hepatic preneoplastic lesions induced by mutagenic carcinogens: contrasted expression of glutathione S-transferase P form and enoyl CoA hydratase. Carcinogenesis 14, 393-398.
Yu, H., West, M., Keon, B.H., Bilter, G.K., Owens, S., Lamerdin, J., Westwick, J.K. (2003). Measuring drug action in the cellular context using protein-fragment complementation assays. ASSAY and Drug Development Technologies 1, 811-822.
Zabel, U., and Baeuerle, P. A. (1990). Purified human I kappa B can rapidly dissociate the complex of the NF-kappa B transcription factor with its cognate DNA. Cell 61, 255-265.
Zaccolo and T. Pozzan, 2000, Imaging signal transduction in living cells with GFP-based probes, IUBMB Life 49: 1-5,2000.
Zhang, J., Ferguson, S. S., Barak, L. S., Menard, L., and Caron, M. G. (1996). Dynamin and beta-arrestin reveal distinct mechanisms for G protein-coupled receptor internalization. J Biol Chem 271, 18302-18305.

While the many forms of the invention herein disclosed constitute presently preferred embodiments, many others are possible and further details of the preferred embodiments and other possible embodiments are not to be construed as limitations. It is understood that the terms used herein are merely descriptive rather than limiting and that various changes many equivalents may be made without departing from the spirit or scope of the claimed invention.

## Claims

1. A collection of cells or a cell line, wherein said cells or cell line contains a first protein and a second protein, wherein said first protein is operably linked to first fragment of a rationally-dissected reporter and said second protein is operably linked to a second fragment of a rationally-dissected reporter,
wherein the first fragment and the second fragment of said rationally-dissected reporter are complementary mutant fragments of the reporter protein YFP,
wherein said first mutant reporter fragment IFP[1] corresponds to YFP[1] comprising YFP amino acid residues 1-158 and said second mutant reporter fragment IFP[2] corresponds to YFP[2] comprising YFP amino acid residues 159-239,
wherein said first mutant fragment IFP[1] corresponds to YFP[1]-(F46L, F64L, M153T) and said second mutant fragment IFP[2] corresponds to YFP[2]-(V163A, S175G).

2. A collection of cells or a cell line according to claim 1, wherein IFP[1] and IFP [2] incorporate restriction sites and a linker.

3. A collection of cells or a cell line according to claim 2, wherein said linker enodes a flexible 10 amino acid peptide.

4. A collection of cells or a cell line according to claim 3, wherein said flexible 10 amino acid peptide is the peptide (Gly.Gly.Gly.Gly.Ser)2.

5. A collection of cells or a cell line according to any of claims 1-4, wherein said first protein and said second protein are linked either at the 5'- or 3'-end of each reporter fragment.

6. A collection of cells or a cell line according to any of claims 1-5, wherein said first protein and said second protein are subcloned into a mammalian expression vector that incorporates the replication origin of the Epstein Barr virus and that retains the SV40 origin.

7. A collection of cells or a cell line according to any of claims 1-6, wherein said cells or said cell line is selected from the group consisting of HEK293E, HEK293T, Jurkat and COS cells.
